# EUROPEAN PATENT APPLICATION

(11) **EP 3 257 951 A2**
(43) Date of publication of application: **20.12.2017**
(21) Application number: 17176382.4
(22) Date of filing: 16.06.2017
(51) Int. Cl.: C12Q 1/68

(54) **METHODS AND COMPOSITIONS FOR IDENTIFYING AND QUANTIFYING FUNGAL DNA**

(30) Priority: 17.06.2016 US 201662351654 P
(71) Applicant: AdvaTect Diagnostics, LLC, Carrollton, TX 75010 (US)
(72) Inventor: HOOPER, Dennis G., Lewisville, TX 75056 (US); SUTTON, John S., Carrollton, TX 75010 (US)
(74) Representative: Grünecker Patent- und Rechtsanwälte PartG mbB

(57) **Abstract**

This invention relates to methods and compositions for identifying microbial DNA in the tissues or body fluid samples of patients. More particularly, the invention relates to two-step polymerase chain reaction based methods for identifying microbial DNA in the tissues or body fluid samples of patients, and compositions therefor. Microbial DNA can also be quantified using the methods described herein.

## Description

### FIELD OF THE DISCLOSURE

This invention relates to methods and compositions for identifying microbial DNA in the tissues or body fluid samples of patients. More particularly, the invention relates to two-step polymerase chain reaction based methods for identifying microbial DNA in the tissues or body fluid samples of patients, and compositions therefor. Microbial DNA can also be quantified using the methods described herein.

### BACKGROUND AND SUMMARY

Molds (*i.e.*, toxigenic and other septate molds) are ubiquitous in the environment. Mold is the common name for various types of fungi. Molds are usually found in moist, warm environments. Because molds grow in wet or moist indoor environments, people are exposed to molds or their byproducts through either direct contact, or through the air, if molds or mold byproducts are aerosolized. Exposure to molds can cause a number of adverse effects including allergic reactions, asthma attacks, and infections, particularly in individuals with immune system deficiencies.

Adverse effects from molds may occur when individuals are exposed to large doses of chemicals, known as mycotoxins, which are fungal metabolites (Samson et al., 1985; Burge, 1990; Flannigan et al., 1991). Mycotoxins have toxic effects ranging from severe irritations, such as allergic reactions and asthma, to immuno-suppression and cancer. Most mycotoxins are cytotoxic and exert their effects by interfering with vital cellular processes such as protein, RNA, and DNA synthesis. As a result, mycotoxins may be damaging to the skin, the lungs, the gut, and the like. The combined outcome may increase the susceptibility of the exposed individual to infectious diseases and, possibly, to cancer. Almost all of the studies to date focus on disease induced by mycotoxins ingested in contaminated food (Baxter et al., 1981), but mycotoxins are secondary metabolites of fungal spores and can enter the body through the respiratory tract.

In heavily contaminated environments, neurotoxic symptoms related to airborne mycotoxin exposure have been reported (Croft et al., 1986). Skin is another potential route of exposure to the mycotoxins of several fungi which have caused cases of severe dermatosis (Vennewald and Wollina, 2005). These same molds may cause invasive mold infection among patients with diseases which render the patient immuno-suppressed such as leukemia, lymphoma, and many cancers (Kontoyiannis, DP et al, 2005). The mold infections in such patients are often fatal with a documented fatally rate of 92% (Paterson and Singh, 1999).

A definitive and early diagnosis of a fungal infection is crucial for patient treatment and management. Several reasons for the late diagnosis of fungal infections include the lack of good clinical specimens, the difficultly in differentiating invasive mold infections from other types of infections, the lack of identification of molds with special stains in pathological specimens (*i.e.,* these assays have a high error rate, a low sensitivity, and low specificity), and the lack of an ability to obtain an antibody-based diagnosis in immuno-compromised patients.

Thus, reliable, sensitive, specific, and rapid methods for mold detection and/or quantitation in patient body fluids and tissues are needed. Applicant's present invention is based on the development of reliable, sensitive, specific, and rapid methods for detecting fungal DNA, and other microbial DNA, in patient body fluids and tissues to determine the cause of diseases related to infections (e.g., mold infections) so that effective treatment regimens can be developed.

In one embodiment a method of identifying a specific fungal species in a patient tissue or a patient body fluid is provided. The method comprises extracting and recovering fungal DNA of the fungal species from the patient tissue or the patient body fluid, amplifying the fungal DNA using a first set of primers to produce an amplicon, amplifying the amplicon using a second set of primers, hybridizing an isolated probe to the amplicon to identify the specific fungal species, wherein the probe is labeled with at least one fluorescent dye, and identifying the specific fungal species.

In another illustrative embodiment, a method is provided of determining if a patient is at risk for or has developed a disease state related to a fungal infection. The method comprises extracting and recovering fungal DNA of the fungal species from the patient tissue or the patient body fluid, amplifying the fungal DNA using a first set of primers to produce an amplicon, amplifying the amplicon using a second set of primers, hybridizing an isolated probe to the amplicon to identify the specific fungal species, wherein the probe is labeled with at least one fluorescent dye, identifying the specific fungal species to determine if the patient is at risk for or has developed the disease state related to a fungal infection.

In still another embodiment, a kit is provided. The kit comprises a purified nucleic acid with a sequence selected from SEQ ID NOS: 1 to 18, 55 and 56, or with a complement of a sequence selected from SEQ ID NOS: 1 to 18, 55, and 56, and a fluorescently labeled probe.

In yet another aspect, a purified nucleic acid is provided with a sequence selected from SEQ ID NOS: 1 to 18, 55, and 56, in combination with fluorescently labeled target DNA.

In another embodiment, a purified nucleic acid is provided that hybridizes under highly stringent conditions to a sequence selected from SEQ ID NOS: 1 to 18, 55, and 56, in combination with fluorescently labeled target DNA.

In another aspect, a purified nucleic acid is provided comprising a complement of a sequence selected from SEQ ID NOS: 1 to 18, 55, and 56, in combination with fluorescently labeled target DNA.

Several additional embodiments of the invention are described in the following enumerated clauses. Any combination of the following embodiments is also contemplated along with any applicable combination with the embodiments described in the DETAILED DESCRIPTION OF ILLUSTRATIVE EMBODIMENTS section of this patent application.
1. A method of identifying a specific fungal species in a patient tissue or a patient body fluid, the method comprising,
   extracting and recovering fungal DNA of the fungal species from the patient tissue or the patient body fluid,
   amplifying the fungal DNA using a first set of primers to produce an amplicon,
   amplifying the amplicon using a second set of primers,
   hybridizing an isolated probe to the amplicon to identify the specific fungal species, wherein the probe is labeled with at least one fluorescent dye, and
   identifying the specific fungal species.
2. The method of clause 1 wherein the amplifying steps are performed with primers that hybridize to the fungal DNA or to the amplicon.
3. The method of clause 1 or 2 wherein the body fluid is selected from the group consisting of urine, nasal secretions, nasal washes, bronchial lavages, bronchial washes, spinal fluid, sputum, gastric secretions, seminal fluid, other reproductive tract secretions, lymph fluid, whole blood, blood from a blood card, serum, buffy coat, and plasma.
4. The method of any one of clauses 1 to 3 wherein the fungal DNA is amplified using PCR.
5. The method of any one of clauses 1 to 4 wherein the amplicon is amplified using PCR.
6. The method of clause 4 wherein the PCR is real-time PCR.
7. The method of clause 5 wherein the PCR is real-time PCR.
8. The method of any one of clauses 1 to 7 wherein the fungal species is selected from the group consisting of *Aspergillus niger*, *Aspergillus flavus*, *Aspergillus fumigatus, Aspergillus terreus*, *Candida albicans, Candida auris*, *Candida tropicalis, Candida glabrata, Candida krusei,* and *Candida parapsilosis.*
9. The method of any one of clauses 1 to 8 wherein the first set of primers is selected from the group consisting of SEQ ID NOS:1 and 2, SEQ ID NOS:3 and 4, SEQ ID NOS:5 and 6, SEQ ID NOS:7 and 8, SEQ ID NOS:9 and 10, SEQ ID NOS:11 and 12, SEQ ID NOS: 13 and 14, SEQ ID NOS:15 and 16, SEQ ID NOS: 17 and 18, and SE ID NOS: 55 and 56.
10. The method any one of clauses 1 to 9 wherein the second set of primers is selected from the group consisting of SEQ ID NOS:20 and 21, SEQ ID NOS:23 and 24, SEQ ID NOS:26 and 27, SEQ ID NOS:29 and 30, SEQ ID NOS:32 and 33, SEQ ID NOS:35 and 36, SEQ ID NOS:38 and 39, SEQ ID NOS:41 and 42, SEQ ID NOS:44 and 45, SEQ ID NOS:47 and 48, and SEQ ID NOS: 58 and 59.
11. The method of any one of clauses 1 to 10 wherein the probe is selected from the group consisting of SEQ ID NOS: 19, 22, 25, 28, 31, 34, 37, 40, 43, 46, and 57.
12. The method of any one of clauses 1 to 11 wherein the fungal DNA or the amplicon is from internal transcribed spacer regions of nuclear ribosomal DNA.
13. The method of any one of clauses 1 to 12 wherein the at least one fluorescent dye comprises a fluorescence resonance energy transfer pair.
14. The method of any one of clauses 1 to 13 wherein the at least one fluorescent dye comprises FAM.
15. The method of any one of clauses 1 to 14 wherein the at least one fluorescent dye comprises dCAL FLUOR Orange 560.
16. The method of any one of clauses 1 to 15 wherein the at least one fluorescent dye comprises BHQ.
17. The method of any one of clauses 1 to 16 further comprising quantifying the fungal DNA.
18. The method of any one of clauses 1 to 17 wherein the method can be used to detect 0.1 ng of the fungal DNA.
19. A method of determining if a patient is at risk for or has developed a disease state related to a fungal infection, the method comprising,
   extracting and recovering fungal DNA of the fungal species from the patient tissue or the patient body fluid,
   amplifying the fungal DNA using a first set of primers to produce an amplicon,
   amplifying the amplicon using a second set of primers,
   hybridizing an isolated probe to the amplicon to identify the specific fungal species, wherein the probe is labeled with at least one fluorescent dye,
   identifying the specific fungal species to determine if the patient is at risk for or has developed the disease state related to a fungal infection.
20. The method of clause 19 wherein the amplifying steps are performed with primers that hybridize to the fungal DNA or to the amplicon.
21. The method of clause 19 or 20 wherein the body fluid is selected from the group consisting of urine, nasal secretions, nasal washes, bronchial lavages, bronchial washes, spinal fluid, sputum, gastric secretions, seminal fluid, other reproductive tract secretions, lymph fluid, whole blood, blood from a blood card, serum, buffy coat, and plasma.
22. The method of any one of clauses 19 to 21 wherein the fungal DNA is amplified using PCR.
23. The method of any one of clauses 19 to 22 wherein the amplicon is amplified using PCR.
24. The method of clause 22 wherein the PCR is real-time PCR.
25. The method of clause 23 wherein the PCR is real-time PCR.
26. The method of any one of clauses 19 to 25 wherein the fungal species is selected from the group consisting of *Aspergillus niger*, *Aspergillus flavus*, *Aspergillus fumigatus, Aspergillus terreus, Candida albicans, Candida auris*, *Candida tropicalis, Candida glabrata, Candida krusei,* and *Candida parapsilosis.*
27. The method of any one of clauses 19 to 26 wherein the first set of primers is selected from the group consisting of SEQ ID NOS:1 and 2, SEQ ID NOS:3 and 4, SEQ ID NOS:5 and 6, SEQ ID NOS:7 and 8, SEQ ID NOS:9 and 10, SEQ ID NOS:11 and 12, SEQ ID NOS:13 and 14, SEQ ID NOS:15 and 16, SEQ ID NOS: 17 and 18, and SEQ ID NOS: 55 and 56.
28. The method of any one of clauses 19 to 27 wherein the second set of primers is selected from the group consisting of SEQ ID NOS:20 and 21, SEQ ID NOS:23 and 24, SEQ ID NOS:26 and 27, SEQ ID NOS:29 and 30, SEQ ID NOS:32 and 33, SEQ ID NOS:35 and 36, SEQ ID NOS:38 and 39, SEQ ID NOS:41 and 42, SEQ ID NOS:44 and 45, SEQ ID NOS:47 and 48, and SEQ ID NOS: 58 and 59.
29. The method of any one of clauses 19 to 28 wherein the probe is selected from the group consisting of SEQ ID NOS: 19, 22, 25, 28, 31, 34, 37, 40, 43, 46, and 57.
30. The method of any one of clauses 19 to 29 wherein the fungal DNA or the amplicon is from internal transcribed spacer regions of nuclear ribosomal DNA.
31. The method of any one of clauses 19 to 30 wherein the at least one fluorescent dye comprises a fluorescence resonance energy transfer pair.
32. The method of any one of clauses 19 to 31 wherein the at least one fluorescent dye comprises FAM.
33. The method of any one of clauses 19 to 32 wherein the at least one fluorescent dye comprises dCAL FLUOR Orange 560.
34. The method of any one of clauses 19 to 33 wherein the at least one fluorescent dye comprises BHQ.
35. The method of any one of clauses 19 to 34 further comprising quantifying the fungal DNA.
36. The method of any one of clauses 19 to 35 wherein the method can be used to detect 0.1 ng of the fungal DNA.
37. The method of any one of clauses 19 to 36 further comprising developing an effective treatment regimen for the patient.
39. A kit comprising a purified nucleic acid with a sequence selected from SEQ ID NOS: 1 to 18, 55 and 56, or with a complement of a sequence selected from SEQ ID NOS: 1 to 18, 55, and 56, and a fluorescently labeled probe.
40. A purified nucleic acid with a sequence selected from SEQ ID NOS: 1 to 18, 55, and 56 in combination with fluorescently labeled target DNA.
41. A purified nucleic acid that hybridizes under highly stringent conditions to a sequence selected from SEQ ID NOS: 1 to 18, 55, and 56 in combination with fluorescently labeled target DNA.
42. A purified nucleic acid comprising a complement of a sequence selected from SEQ ID NOS: 1 to 18, 55, and 56 in combination with fluorescently labeled target DNA.
43. The method of any one of clauses 1 to 37 further comprising identifying a mycotoxin in the patient tissue or body fluid.
44. The method of any one of clauses 1 to 37 wherein the fungal DNA is amplified in a separate reaction vessel than the amplicon.
45. The method of any one of clauses 1 to 37,43, or 44 wherein the fungal DNA is detected by amplifying the fungal DNA using the first set of primers to produce the amplicon, and amplifying the amplicon using the second set of primers, but is not detected using a single real-time PCR amplification step.

### BRIEF DESCRIPTION OF THE DRAWINGS

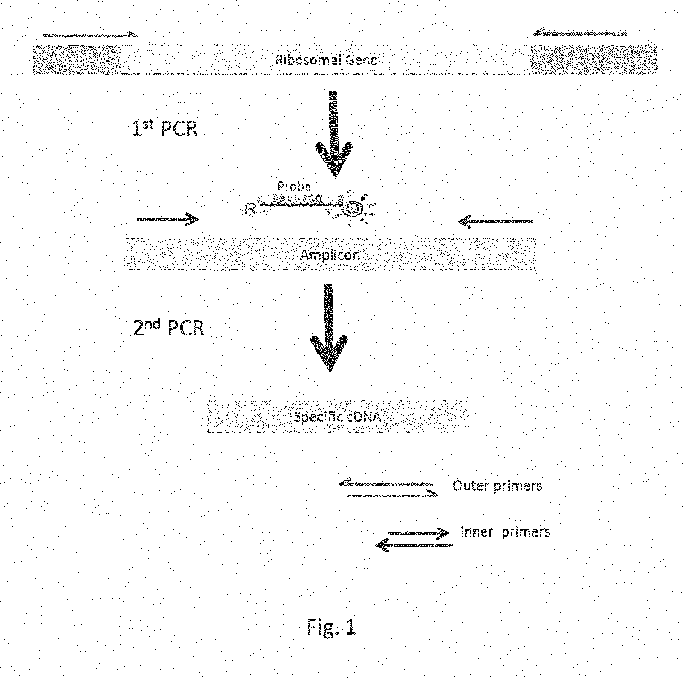

Fig. 1 is a diagram of DNA testing by a two-step PCR method described herein.

### DESCRIPTION OF THE ILLUSTRATIVE EMBODIMENTS

The present invention relates to methods for identifying or detecting the presence of molds (*i.e.*, fungi) in patient tissue and body fluids. The identification and detection methods are based on amplification of fungal DNA using polymerase chain reaction (PCR)-based methods. The methods and compositions (e.g., primers and probes) for amplification of fungal DNA are specific and sensitive and avoid co-amplification of or do not co-amplify non-specific human or animal nucleic acids.

Several additional embodiments of the invention are described in the following enumerated clauses. Any combination of the following embodiments is also contemplated along with any applicable combination with the other embodiments described in this DETAILED DESCRIPTION OF ILLUSTRATIVE EMBODIMENTS section of this patent application.
1. A method of identifying a specific fungal species in a patient tissue or a patient body fluid, the method comprising,
   extracting and recovering fungal DNA of the fungal species from the patient tissue or the patient body fluid,
   amplifying the fungal DNA using a first set of primers to produce an amplicon,
   amplifying the amplicon using a second set of primers,
   hybridizing an isolated probe to the amplicon to identify the specific fungal species, wherein the probe is labeled with at least one fluorescent dye, and identifying the specific fungal species.
2. The method of clause 1 wherein the amplifying steps are performed with primers that hybridize to the fungal DNA or to the amplicon.
3. The method of clause 1 or 2 wherein the body fluid is selected from the group consisting of urine, nasal secretions, nasal washes, bronchial lavages, bronchial washes, spinal fluid, sputum, gastric secretions, seminal fluid, other reproductive tract secretions, lymph fluid, whole blood, blood from a blood card, serum, buffy coat, and plasma.
4. The method of any one of clauses 1 to 3 wherein the fungal DNA is amplified using PCR.
5. The method of any one of clauses 1 to 4 wherein the amplicon is amplified using PCR.
6. The method of clause 4 wherein the PCR is real-time PCR.
7. The method of clause 5 wherein the PCR is real-time PCR.
8. The method of any one of clauses 1 to 7 wherein the fungal species is selected from the group consisting of *Aspergillus niger*, *Aspergillus flavus*, *Aspergillus fumigatus*, *Aspergillus terreus, Candida albicans, Candida auris*, *Candida tropicalis, Candida glabrata, Candida krusei,* and *Candida parapsilosis.*
9. The method of any one of clauses 1 to 8 wherein the first set of primers is selected from the group consisting of SEQ ID NOS:1 and 2, SEQ ID NOS:3 and 4, SEQ ID NOS:5 and 6, SEQ ID NOS:7 and 8, SEQ ID NOS:9 and 10, SEQ ID NOS:11 and 12, SEQ ID NOS:13 and 14, SEQ ID NOS:15 and 16, SEQ ID NOS: 17 and 18, and SEQ ID NOS: 55 and 56.
10. The method any one of clauses 1 to 9 wherein the second set of primers is selected from the group consisting of SEQ ID NOS:20 and 21, SEQ ID NOS:23 and 24, SEQ ID NOS:26 and 27, SEQ ID NOS:29 and 30, SEQ ID NOS:32 and 33, SEQ ID NOS:35 and 36, SEQ ID NOS:38 and 39, SEQ ID NOS:41 and 42, SEQ ID NOS:44 and 45, SEQ ID NOS:47 and 48, and SEQ ID NOS: 59 and 59.
11. The method of any one of clauses 1 to 10 wherein the probe is selected from the group consisting of SEQ ID NOS: 19, 22, 25, 28, 31, 34, 37, 40, 43, 46, and 57.
12. The method of any one of clauses 1 to 11 wherein the fungal DNA or the amplicon is from internal transcribed spacer regions of nuclear ribosomal DNA.
13. The method of any one of clauses 1 to 12 wherein the at least one fluorescent dye comprises a fluorescence resonance energy transfer pair.
14. The method of any one of clauses 1 to 13 wherein the at least one fluorescent dye comprises FAM.
15. The method of any one of clauses 1 to 14 wherein the at least one fluorescent dye comprises dCAL FLUOR Orange 560.
16. The method of any one of clauses 1 to 15 wherein the at least one fluorescent dye comprises BHQ.
17. The method of any one of clauses 1 to 16 further comprising quantifying the fungal DNA.
18. The method of any one of clauses 1 to 17 wherein the method can be used to detect 0.1 ng of the fungal DNA.
19. A method of determining if a patient is at risk for or has developed a disease state related to a fungal infection, the method comprising,
   extracting and recovering fungal DNA of the fungal species from the patient tissue or the patient body fluid,
   amplifying the fungal DNA using a first set of primers to produce an amplicon,
   amplifying the amplicon using a second set of primers,
   hybridizing an isolated probe to the amplicon to identify the specific fungal species, wherein the probe is labeled with at least one fluorescent dye,
   identifying the specific fungal species to determine if the patient is at risk for or has developed the disease state related to a fungal infection.
20. The method of clause 19 wherein the amplifying steps are performed with primers that hybridize to the fungal DNA or to the amplicon.
21. The method of clause 19 or 20 wherein the body fluid is selected from the group consisting of urine, nasal secretions, nasal washes, bronchial lavages, bronchial washes, spinal fluid, sputum, gastric secretions, seminal fluid, other reproductive tract secretions, lymph fluid, whole blood, blood from a blood card, serum, buffy coat, and plasma.
22. The method of any one of clauses 19 to 21 wherein the fungal DNA is amplified using PCR.
23. The method of any one of clauses 19 to 22 wherein the amplicon is amplified using PCR.
24. The method of clause 22 wherein the PCR is real-time PCR.
25. The method of clause 23 wherein the PCR is real-time PCR.
26. The method of any one of clauses 19 to 25 wherein the fungal species is selected from the group consisting of *Aspergillus niger*, *Aspergillus flavus, Aspergillus fumigatus, Aspergillus terreus, Candida albicans, Candida auris*, *Candida tropicalis, Candida glabrata, Candida krusei,* and *Candida parapsilosis.*
27. The method of any one of clauses 19 to 26 wherein the first set of primers is selected from the group consisting of SEQ ID NOS:1 and 2, SEQ ID NOS:3 and 4, SEQ ID NOS:5 and 6, SEQ ID NOS:7 and 8, SEQ ID NOS:9 and 10, SEQ ID NOS:11 and 12, SEQ ID NOS: 13 and 14, SEQ ID NOS: 15 and 16, SEQ ID NOS: 17 and 18, and SEQ ID NOS: 55 and 56.
28. The method of any one of clauses 19 to 27 wherein the second set of primers is selected from the group consisting of SEQ ID NOS:20 and 21, SEQ ID NOS:23 and 24, SEQ ID NOS:26 and 27, SEQ ID NOS:29 and 30, SEQ ID NOS:32 and 33, SEQ ID NOS:35 and 36, SEQ ID NOS:38 and 39, SEQ ID NOS:41 and 42, SEQ ID NOS:44 and 45, SEQ ID NOS:47 and 48, and SEQ ID NOS: 58 and 59.
29. The method of any one of clauses 19 to 28 wherein the probe is selected from the group consisting of SEQ ID NOS: 19, 22, 25, 28, 31, 34, 37, 40, 43, 46, and 57.
30. The method of any one of clauses 19 to 29 wherein the fungal DNA or the amplicon is from internal transcribed spacer regions of nuclear ribosomal DNA.
31. The method of any one of clauses 19 to 30 wherein the at least one fluorescent dye comprises a fluorescence resonance energy transfer pair.
32. The method of any one of clauses 19 to 31 wherein the at least one fluorescent dye comprises FAM.
33. The method of any one of clauses 19 to 32 wherein the at least one fluorescent dye comprises dCAL FLUOR Orange 560.
34. The method of any one of clauses 19 to 33 wherein the at least one fluorescent dye comprises BHQ.
35. The method of any one of clauses 19 to 34 further comprising quantifying the fungal DNA.
36. The method of any one of clauses 19 to 35 wherein the method can be used to detect 0.1 ng of the fungal DNA.
37. The method of any one of clauses 19 to 36 further comprising developing an effective treatment regimen for the patient.
39. A kit comprising a purified nucleic acid with a sequence selected from SEQ ID NOS: 1 to 18, 55, and 56, or with a complement of a sequence selected from SEQ ID NOS: 1 to 18, 55, and 56, and a fluorescently labeled probe.
40. A purified nucleic acid with a sequence selected from SEQ ID NOS: 1 to 18, 55, and 56 in combination with fluorescently labeled target DNA.
41. A purified nucleic acid that hybridizes under highly stringent conditions to a sequence selected from SEQ ID NOS: 1 to 18, 55, and 56 in combination with fluorescently labeled target DNA.
42. A purified nucleic acid comprising a complement of a sequence selected from SEQ ID NOS: 1 to 18, 55, and 56 in combination with fluorescently labeled target DNA.
43. The method of any one of clauses 1 to 37 further comprising identifying a mycotoxin in the patient tissue or body fluid.
44. The method of any one of clauses 1 to 37 wherein the fungal DNA is amplified in a separate reaction vessel than the amplicon.
45. The method of any one of clauses 1 to 37, 43, or 44 wherein the fungal DNA is detected by amplifying the fungal DNA using the first set of primers to produce the amplicon, and amplifying the amplicon using the second set of primers, but is not detected using a single real-time PCR amplification step.

In various illustrative embodiments, body fluids that can be tested for the presence of fungal DNA, include, but are not limited to, urine, nasal secretions, nasal washes, inner ear fluids, bronchial lavages, bronchial washes, alveolar lavages, spinal fluid, bone marrow aspirates, sputum, pleural fluids, synovial fluids, pericardial fluids, peritoneal fluids, saliva, tears, gastric secretions, stool, reproductive tract secretions, such as seminal fluid, lymph fluid, and whole blood, blood from a blood card, buffy coat, serum, or plasma. These samples can be prepared for testing as described herein. In various embodiments, tissue samples can include tissue biopsies of hospital patients or out-patients and autopsy specimens. As used herein, the term "tissue" includes, but is not limited to, biopsies, autopsy specimens, cell extracts, tissue sections, aspirates, tissue swabs, and fine needle aspirates.

As used herein, the word "patient" means a human or an animal, such as a domestic animal (*e.g*., a dog or a cat). Accordingly, the methods and compositions disclosed herein can be used for both human clinical medicine and veterinary applications. In one aspect, the patient afflicted with a disease state related to a fungal infection can be a human, or in the case of veterinary applications, can be a laboratory, agricultural, domestic or wild animal. In one embodiment, the methods and compositions described herein can be applied to patients including, but not limited to, humans, laboratory animals such as rodents (*e.g*., mice, rats, hamsters, etc.), rabbits, monkeys, chimpanzees, domestic animals such as dogs, cats, and rabbits, agricultural animals such as cows, horses, pigs, sheep, goats, chickens, and wild animals in captivity such as bears, pandas, lions, tigers, leopards, elephants, zebras, giraffes, gorillas, dolphins, and whales.

In various embodiments, the methods and compositions described herein can be used to detect or identify microbial DNA (e.g., fungal DNA). As used herein, the terms "fungal," "fungi," and "fungus" include yeasts. In illustrative embodiments, the DNA that can be identified or detected includes DNA from microbes selected from the group consisting of *Absidia coerulea, Absidia glauca, Absidia corymbifera*, *Acremonium strictum, Alternaria alternarta, Apophysomyces elegans*, *Saksena vasiformis, Aspergillus flavus, Aspergillus oryzae*, *Aspergillus fumigatus, Neosartoryta fischeri, Aspergillus niger*, *Aspergillus foetidus, Aspergillus phoenictus*, *Aspergillus nomius, Aspergillus ochraceus, Aspergillus ostiamus, Aspergillus auricomus, Aspergillus parasiticus, Aspergillus sojae, Aspergillus restrictus, Aspergillus caesillus*, *Aspergillus conicus, Aspergillus sydowii, Aspergillus tamarii, Aspergillus terreus, Aspergillus ustus, Aspergillus versicolor, Aspergillus ustus, Aspergillus versicolor*, *Candida albicans, Candida auris*, *Candida tropicalis, Candida glabrata, Candida krusei, Candida parapsilosis, Chaetomium globosum, Cladosporium cladosporioides, Cladosporium herbarum, Cladosporium sphaerospermum, Conidiobolus coronatus*, *Conidiobolus incongruus, Cunninghamella elegans, Emericella nidulans, Emericella rugulosa*, *Emericilla quadrilineata, Apicoccum nigrum, Eurotium amstelodami, Eurotium chevalieri*, *Eurotium herbariorum, Eurotium rubrum, Eurotium repens, Geometrica candidum, Geotrichum klebahmii. Memnoniella echinata, Mortierella polycephala, Mortierella wolfii, Mucor mucedo, Mucor amphibiorum*, *Mucor circinelloides, Mucor heimalis*, *Mucor indicus*, *Mucor racemosus*, *Mucor ramosissimus*, *Rhizopus azygosporous*, *Rhizopus homothalicus, Rhizopus microsporus, Rhizopus oligosporus, Rhizopus oryzae, Myrothecium verrucaria*, *Myrothecium roridum, Paecilomyces lilacinus, Paecilomyces variotii, Penicillium freii, Penicillium verrucosum, Penicillium hirsutum, Penicillium alberechii, Penicillum aurantiogriseum, Penicillium polonicum. Penicillium viridicatum, Penicillium hirsutum, Penicillium brevicompactum, Penicillium chrysogenum, Penicillium griseofulvum, Penicillium glandicola, Penicillium coprophilum, Eupenicillium crustaceum, Eupenicillium egyptiacum, Penicillium crustosum*, *Penicillium citrinum, Penicillium sartoryi*, *Penicillium westlingi, Penicillium corylophilum, Penicillium decumbens, Penicillium echinulatum, Penicillium solitum, Penicillium camembertii, Penicillium commune*, *Penicillium echinulatum, Penicillium scleroligenum, Penicillium italicum, Penicillium expansum*, *Penicillium fellutanum, Penicillium charlesii, Penicillium janthinellum, Penicillium raperi*, *Penicillium madriti, Penicillium gladioli, Penicillium oxalicum, Penicillium roquefortii, Penicillium simplicissimum*, *Penicillium ochrochloron*, *Penicillium spinulosum, Penicillium glabrum, Penicillium thomii, Penicillium pupurescens, Eupenicillium lapidosum, Rhizomucor miehei, Rhizomucor pusillus, Rhizomucor variabilis. Rhizopus stolonifer, Scopulariopsis asperula*, *Scopulariopsis brevicaulis*, *Scopulariopsis fusca, Scopulariopsis brumptii, Scopulariopsis chartarum, Scopulariopsis sphaerospora, Trichoderma asperellum, Trichoderma hamatum, Trichoderma viride*, *Trichoderma harziamum, Trichoderma longibrachiatum, Trichoderma citroviride, Trichoderma atroviride, Trichoderma koningii*, *Ulocladium atrum, Ulocladium chartarum, Ulocladium botrytis, Wallemia sebi, Stachybotrys chartarum,* and the like.

In embodiments where the microbe is a fungal species, the microbe is typically selected from the group consisting of *S*. *chartarum, S. prolificans, A. versicolor*, *A. vesicularis, A. niger*, *A. terreus, P. chrysogenum, P. verrucosum, G. candidum, A. flavus, A. fumigatus, A. nidulans, A. ochraceus*, *A. paraciticus, A. sydowii, A. ustus, Candida albicans, Candida auris*, *Candida tropicalis, Candida glabrata*, *Candida krusei. C. parapsilosis. F. solani, F. chlamydosporum, Geometrica candidum, P. aurantiogriseum, P. citrinum, P. corylophilum, P. crustosum, P. expansum, P. fellutanum, P. roquefortii*, *P. simplicissimum, S. echinata,* and *E. amstelodami*. In one embodiment, the molds (*i.e*., fungi) can be black, toxigenic molds. In another embodiment where the microbe is a fungal species, the microbe is selected from the group consisting of *A. niger*, *A. terreus, G. candidum, A. flavus, A. fumigatus, Candida albicans, Candida auris*, *Candida tropicalis, Candida glabrata, Candida krusei,* and *Candida parapsilosis.*

In some embodiments, real-time PCR-based methods can be used to amplify the fungal DNA and to detect and identify fungal DNA. In other embodiments, traditional PCR can be used. In one aspect, the amplification of the fungal DNA is performed using traditional PCR and the amplification of the amplicon is performed using real-time PCR. In another embodiment, the amplification of the fungal DNA and the amplification of the amplicon are both performed using real-time PCR. In one aspect of the methods described herein, the amplification of the fungal DNA is performed using traditional PCR (e.g., without a labeled probe or labeled target DNA) and the amplification of the amplicon is performed using real-time PCR. PCR is described in U.S. Patent Nos. 4,683,202 and 4,800,159, incorporated herein by reference, and methods for real-time and traditional PCR are well-known in the art. In one illustrative aspect, real-time PCR combines amplification and simultaneous monitoring of the amplication by, for example, detection of increases in fluorescence, such as with SYBR® Green to achieve sensitive and specific detection of microbial DNA (e.g., fungal DNA) in real-time thereby providing instant detection of microbial DNA (e.g., fungal DNA). In this embodiment, the time to detect or identify the fungus and to obtain a diagnosis is greatly reduced. A diagram depicting an embodiment of a method described herein is shown in Figure 1. In this embodiment, primers with sequences selected from SEQ ID NOS: 1 to 18, 55, and 56 are used to amplify fungal DNA in a first PCR step, and probes and primers with sequences selected from SEQ ID NOS: 19 to 48 and 57 to 59 are used to amplify and detect, in a second PCR step, the amplicon that results from the first PCR step. In this embodiment, the first PCR step and the second PCR step can be performed in different reaction vessels. In another embodiment, primers with sequences selected from SEQ ID NOS: 60 and 61 are used to amplify fungal DNA in a first PCR step, and probes and primers with sequences selected from SEQ ID NOS: 62 to 64 are used to amplify and detect, in a second PCR step, the amplicon that results from the first PCR step. In this embodiment, the first PCR step and the second PCR step can be performed in different reaction vessels. Exemplary primers and their target DNAs that can be used in the first PCR step are shown below. Primer "NF" and "Primer NR" refer to a forward primer and a reverse primer, respectively.

### Target - Aspergillus niger

Primer NF1 5'-aggaagtaaaagtcgtaacaag (SEQ ID NO: 1)
Primer NR1 5'-cgcatttcgctgcgttcttc (SEQ ID NO: 2)

### Target - Aspergillus flavus

Primer NF1 5'-aggaagtaaaagtcgtaacaag (SEQ ID NO: 3)
Primer NR1 5'-cgcatttcgctgcgttcttc (SEQ ID NO: 4)

### Target - Aspergillus fumigatus

Primer NF1 5'-aggaagtaaaagtcgtaacaag (SEQ ID NO: 5)
Primer NR1 5'-cgcatttcgctgcgttcttc (SEQ ID NO: 6)

### Target - Aspergillus terreus

Primer NFAT 5'-gactattgtaccttgttgcttc (SEQ ID NO: 7)
Primer NRAT 5'-cattagttatcgcatttcgctg (SEQ ID NO: 8)

### Target - Candida albicans

Primer NFCA 5'- tagcgaacaagtacagtgatg (SEQ ID NO: 9)
Primer NRCA 5'-ctcggtctaggctggcag (SEQ ID NO: 10)

### Target - Candida tropicalis

Primer NFCT 5'-atggaaagatgaaaagaactttg (SEQ ID NO: 11)
Primer NRCT 5'-gctggcagtatcgacgaag (SEQ ID NO: 12)

### Target - Candida glabrata

Primer NFCG 5'-gcttgggactctcgcag (SEQ ID NO: 13)
Primer NRCG 5'-ggcatataaccattatgccag (SEQ ID NO: 14)

### Target - Candida krusei

Primer NFCK 5'-aaaccaacagggattg (SEQ ID NO: 15)
Primer NRCK 5'-cccaaacaactcgac (SEQ ID NO: 16)

### Target - Candida purapsilosis

Primer NFCP 5'- CGTGAAATTGTTGAAAGGGAAG (SEQ ID NO: 17)
Primer NRCP 5' - CTGGCAGTATCGACAAAGAC (SEQ ID NO: 18)

### Target - Candida auris

Primer NCAUF -5'-GAATCGCTCCGGCGAGTTG (SEQ ID NO: 55)
Primer NCAUR -5'-TGTACTTGTTCGCTATCGGTC (SEQ ID NO: 56)

### Target - Rhizopus/Mucor species

Primer MucorF -5'-TACGTCCCTGCCCTTTGTAC (SEQ ID NO: 60)
Primer MucorR -5'-GGAACCTTGTTACGACTTTTAC (SEQ ID NO: 61)

Exemplary probes and primers and their target DNAs that can be used in the second PCR step are shown below. Primer "F" and "Primer R" refer to a forward primer and a reverse primer, respectively. Other primers and probes that can be used in the second PCR step are described in U.S. Appl. Publication No. 20130183697, incorporated herein by reference.

### Target - Aspergillus niger

Probe niger: 5'-TGTCTATTGTACCCTGTTGCTTC (SEQ ID NO: 19)
Primer F1: 5'-CGTAGGTGAACCTGCGGAAG (SEQ ID NO: 20)
Primer R1: 5'-ATCGATGCCGGAACCAAGAG (SEQ ID NO: 21)

### Target - Geometrica candidum

Probe 6 geo: 5'-AACGCACATTGCACTTTGGGGTATC (SEQ ID NO: 22)
Geo F1H: 5'-GGATCTCTTGGTTCTCGTATC (SEQ ID NO: 23)
Geo R1H: 5'-CTTGATCTGAGGTTGAATAGTG (SEQ ID NO: 24)

### Target - Aspergillus flavus

Probe flav: 5'-CCCGCCATTCATGGCCGCCGGG (SEQ ID NO: 25)
Primer F1: 5'-CGTAGGTGAACCTGCGGAAG (SEQ ID NO: 26)
Primer R1: 5'-ATCGATGCCGGAACCAAGAG (SEQ ID NO: 27)

### Target - Aspergillus fumigatus

Probe fumi: 5'-AAAGTATGCAGTCTGAGTTGATTATC (SEQ ID NO: 28)
Primer F1: 5'-CGTAGGTGAACCTGCGGAAG (SEQ ID NO: 29)
Primer R1: 5'- ATCGATGCCGGAACCAAGAG (SEQ ID NO: 30)

### Target - Aspergillus terreus

Probe : 5'- AGTCTGAGTGTGATTCTTTGCAATC (SEQ ID NO: 31)
Primer F: 5'-ACATGAACCCTGTTCTGAAAG (SEQ ID NO: 32)
Primer R: 5'-CCAAGAGATCCATTGTTGAAAG (SEQ ID NO: 33)

### Target - Candida albicans

Probe CA: 5' -TCGGGGGCGGCCGCTGCGG (SEQ ID NO: 34)
Primer F: CA 5' -AAAAAGTACGTGAAATTGTTG (SEQ ID NO: 35)
Primer R: CA 5' -AAGCCGTGCCACATTC (SEQ ID NO: 36)

### Target - Candida krusei

Probe CK: 5' -AAGGCGGTGTCCAAGTCCCTTG (SEQ ID NO: 37)
Primer F: CK 5' -TCAGTAGCGGCGAGTGAAG (SEQ ID NO: 38)
Primer R: CK 5' -AGAAGGGCCTCACTGCTTC (SEQ ID NO: 39)

### Target - Candida glabrata

Probe CG: 5' -ACCTAGGGAATGTGGCTCTGCG (SEQ ID NO: 40)
Primer F: CG 5' -TGGGCCAGCATCGGTTTTG (SEQ ID NO: 41)
Primer R: CG 5' -CCTAGATAACAAGTATCGCAG (SEQ ID NO: 42)

### Target - Candida tropicalis

Probe CT: 5' -TCGGGGGTGGCCTCTACAG (SEQ ID NO: 43)
Primer F: CT 5' -AAAAAGTACGTGAAATTGTTG (SEQ ID NO: 44)
Primer R: CT 5' - AAGCCGTGCCACATTC (SEQ ID NO: 45)

### Target - Candida parapsilosis

Probe CparP1 : 5'-CCTCTACAGTTTACCGGGCCAGCATCA (SEQ ID NO: 46)
Primer CparF1 : 5'-GATCAGACTTGGTATTTTGTATGTTACTCTC (SEQ ID NO: 47)
Primer CparR1 : 5'-CAGAGCCACATTTCTTTGCAC (SEQ ID NO: 48)

### Target - Candida auris

Probe CAUP -5'-CTGCTTTTGCTAGTGCTTCCTGTG (SEQ ID NO: 57)
Primer CAUF -5'-CGAGGTGTTCTAGCAGCAG (SEQ ID NO: 58)
Primer CAUR -5'-ATTTAGCCTTAGATGGAATTTAC (SEQ ID NO: 59)

### Target - Rhizopus/Mucor species

Probe MucP1-5'-CCGATTGAATGGTTATAGTGAGCATATGGGATC (SEQ ID NO: 62)
Primer NS92F -5'-CACCGCCCGTCGCTAC (SEQ ID NO: 63)
Primer MucR1 -5'-CCTAGTTTGCCATAGTTCTCAGCAG (SEQ ID NO: 64)

In the described embodiments, the Rhizopus/Mucor species primers and probes detect multiple fungal species, including Mucor species (such as Mycocladus sp., including Lichtheimia (Absidia, Mycocladus), amphibiorum, circinelloides, hiemalis, indicus, mucedo, racemosus, ampsissimus), Rhizopus species (such as azygosporus, homothalicus, microsporotus, obligosporus, oryzae), and Rhizomucor species.

In various embodiments, sample preparation (*i.e.*, preparation of the target fungal DNA or extracting the fungal DNA) involves rupturing the cells (e.g., cells of the tissue or fungal spores in patient body fluid or patient tissue) and isolating the DNA from the lysate. Techniques for rupturing cells and for isolation of DNA are well-known in the art. For example, cells may be ruptured by using a detergent or a solvent, such as phenol-chloroform. DNA may be separated from the lysate by physical methods including, but not limited to, centrifugation, pressure techniques, or by using a substance with affinity for DNA, such as, for example, silica beads, or by column purification. After sufficient washing, the isolated DNA may be suspended in either water or a buffer. In other embodiments, commercial kits are available, such as QIAGEN®, NUCLISENSM™, and WIZARD™ (Promega), PROMEGAM™, and QIAAMP® DSP DNA Mini Kit (Qiagen). Methods for isolating DNA are described in Sambrook et al., "Molecular Cloning: A Laboratory Manual", 3rd Edition, Cold Spring Harbor Laboratory Press, (2001), incorporated herein by reference.

In various embodiments described herein, the primers and probes used for amplification of the target DNA (e.g., fungal DNA) and for detection, identification, and quantitation of the target DNA (e.g., fungal DNA) are oligonucleotides from about ten to about one hundred, more typically from about ten to about thirty or about six to about twenty-five base pairs long, but any suitable sequence length can be used. In illustrative embodiments, the primers and probes may be double-stranded or single-stranded, but the primers and probes are typically single-stranded. In one aspect, the primers and probes described herein are capable of specific hybridization, under appropriate hybridization conditions (e.g., appropriate buffer, ionic strength, temperature, formamide, and MgCl₂ concentrations), to a region of the target DNA (e.g., fungal DNA). In one embodiment, the primers and probes described herein are designed based on having a melting temperature within a certain range, and substantial complementarity to the target DNA. Methods for the design of primers and probes are described in Sambrook et al., "Molecular Cloning: A Laboratory Manual", 3rd Edition, Cold Spring Harbor Laboratory Press, (2001), incorporated herein by reference.

In various aspects, the primers and probes described herein for use in PCR can be modified by substitution, deletion, truncation, and/or can be fused with other nucleic acid molecules wherein the resulting primers and probes hybridize specifically to the intended targets and are useful in the methods described herein for amplification of the target DNAs. In other embodiments, derivatives can be made such as phosphorothioate, phosphotriester, phosphoramidate, and methylphosphonate derivatives that specifically bind to single-stranded DNA or RNA (Goodchild, et al., Proc. Natl. Acad. Sci. 83:4143-4146 (1986)).

In one illustrative aspect, the methods and compositions described herein encompass isolated or purified nucleic acids. An "isolated" or "purified" nucleic acid molecule is substantially free of other cellular material or culture medium when produced by recombinant techniques, or substantially free of chemical precursors or other chemicals when chemically synthesized. Preferably, an "isolated" or "purified" nucleic acid is free of sequences that naturally flank the nucleic acid in the genomic DNA of the organism from which the nucleic acid is derived. For example, in various embodiments, the isolated or purified nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived.

In other embodiments, nucleic acids complementary to the probes and primers described herein are contemplated, and those that hybridize to the nucleic acids described herein or those that hybridize to their complements under highly stringent conditions are also contemplated. In accordance with the methods described herein, "highly stringent conditions" means hybridization at 65 °C in 5X SSPE and 50% formamide, and washing at 65 °C in 0.5X SSPE. Conditions for low stringency and moderately stringent hybridization are described in Sambrook et al., "Molecular Cloning: A Laboratory Manual", 3rd Edition, Cold Spring Harbor Laboratory Press, (2001), incorporated herein by reference. In some illustrative aspects, hybridization occurs along the full-length of the nucleic acid.

In other illustrative aspects, nucleic acid molecules having about 60%, about 70%, about 75%, about 80%, about 85%, about 90%, about 95%, 96%, 97%, and 98% homology to the probes and primers described herein can be used in the methods or compositions described herein. Determination of percent identity or similarity between sequences can be done, for example, by using the GAP program (Genetics Computer Group, software; now available via Accelrys on http://www.accelrys.com), and alignments can be done using, for example, the ClustalW algorithm (VNTI software, InforMax Inc.). For example, a sequence database can be searched using the nucleic acid sequence of interest. Algorithms for database searching are typically based on the BLAST software (Altschul et al., 1990). In some embodiments, the percent identity can be determined along the full-length of the nucleic acid.

As used herein, the term "complementary" refers to the ability of purine and pyrimidine nucleotide sequences to associate through hydrogen bonding to form double-stranded nucleic acid molecules. Guanine and cytosine, adenine and thymine, and adenine and uracil are complementary and can associate through hydrogen bonding resulting in the formation of double-stranded nucleic acid molecules when two nucleic acid molecules have "complementary" sequences. In one embodiment, the complementary sequences can be DNA or RNA sequences. The complementary DNA or RNA sequences are referred to as a "complement."

Techniques for synthesizing the probes and primers described herein are well-known in the art and include chemical syntheses and recombinant methods. Such techniques are described in Sambrook et al., "Molecular Cloning: A Laboratory Manual", 3rd Edition, Cold Spring Harbor Laboratory Press, (2001), incorporated herein by reference. Primers and probes can also be made commercially (*e.g*., CytoMol, Sunnyvale, CA or Integrated DNA Technologies, Skokie, IL). Techniques for purifying or isolating the probes and primers described herein are well-known in the art. Such techniques are described in Sambrook et al., ''Molecular Cloning: A Laboratory Manual'', 3rd Edition, Cold Spring Harbor Laboratory Press, (2001), incorporated herein by reference. The primers and probes described herein can be analyzed by techniques known in the art, such as restriction enzyme analysis or sequencing, to determine if the sequence of the primers and probes is correct.

In various embodiments of the methods and compositions described herein, the probes and primers can be labeled, such as with fluorescent compounds, radioactive isotopes, antigens, biotin-avidin, colorimetric compounds, or other labeling agents known to those of skill in the art, to allow detection, indentification, and quantification of amplified DNA, such as by Real-time PCR. In illustrative embodiments, the labels may include 6-carboxyfluorescein (FAM™), TET™ (tetrachloro-6-carboxyfluorescein), JOE™ (2,7, -dimethoxy-4,5-dichloro-6-carboxyfluorescein), VIC™, HEX (hexachloro-6-carboxyfluorescein), TAMRA™ (6-carboxy-N,N,N',N'-tetramethylrhodamine), BHQ®, dCAL™ FLUOR® Orange 560, SYBR® Green, ALEXA FLUOR® 350, ALEXA FLUOR® 430, AMCA, BODIPY® 630/650, BODIPY® 650/665, BODIPY®-FL, BODIPY®-R6G, BODIPY®-TMR, BODIPY®-TRX, Cascade Blue, CY®3, CY®5,6-FAM™, Fluorescein, OREGON GREEN® 488, OREGON GREEN® 500, OREGON GREEN® 514, PACIFIC BLUE™, REG, Rhodamine Green, Rhodamine Red, ROX™, and/or TEXAS RED®.

In one embodiment, the methods and compositions (e.g., primers and probes) for amplification of DNA (e.g., fungal DNA) can be highly specific and avoid co-amplification of or do not co-amplify non-specific nucleic acids. In various embodiments, methods can detect 0.1 ng/ml of DNA, 0.2 ng/ml of DNA, 0.3 ng/ml of DNA, 0.4 ng/ml of DNA, 0.5 ng/ml of DNA, 0.6 ng/ml of DNA, 0.7 ng/ml of DNA, 0.8 ng/ml of DNA, 0.9 ng/ml of DNA, 1.0 ng/ml of DNA, 2.0 ng/ml of DNA, or 3.0 ng/ml of DNA. In one embodiment, the DNA is fungal DNA. In various embodiments, the method described herein may detect as few as 1 copy, 2 copies, 3 copies, 4 copies, 5 copies, 6 copies, 7 copies, 8 copies, 9 copies, 10 copies, 20 copies, 30 copies, 40 copies, 50 copies, 60 copies, 70 copies, 80 copies, 90 copies, 100 copies, 200 copies, 300 copies, 400 copies, 500 copies, 600 copies, 700 copies, 800 copies, 900 copies, or 1000 copies of fungal DNA. In another aspect, the fungal DNA is detected using a two-step PCR method (i.e., two amplification steps), wherein the fungal DNA cannot be detected using a single amplification step (e.g., a single real-time PCR step). In another embodiment, the fungal DNA is detected using two amplification steps (e.g., amplifying the fungal DNA using a first set of primers to produce an amplicon, and amplifying the amplicon using a second set of primers and a probe or other label), but is not detected using a single amplification step (e.g., a single real-time PCR step).

In one illustrative embodiment, universal probes can be used to provide a method for determining the presence of fungal DNA before conducting target-specific assays for a DNA target. In one embodiment, universal probes and primers can be used to detect the presence of *Aspergillus* and *Penicillium* species (see probes and primers for Fungal Universal Group 1 below). In another embodiment, universal probes and primers can be used to detect the presence of *Stachybotrys* and *Fusarium* species (see probes and primers for Fungal Universal Group 2 below). In these embodiments, the probes and primers can be homologous for all targets of interest related to *Aspergillus*, *Penicillium*, *Stachybotrys,* and *Fusarium* species.
Fungal Universal Group 1
   UP1: 5'- cctcggatcaggtagggatac (SEQ ID NO: 49)
   UF1: 5'-atgcctgtccgagcgtcatt (SEQ ID NO: 50)
   UR1: 5'- ttcctccgcttattgatatg (SEQ ID NO: 51)
Fungal Universal Group 2
   Up2: 5'- acggatctcttggctctggcatc (SEQ ID NO: 52)
   F2: 5'- gcggagggatcattaccgag (SEQ ID NO: 53)
   UR2: 5'- ttcactgaattctgcaattcac (SEQ ID NO: 54)

In another illustrative embodiment where fungal DNA is identified, the methods described herein can be used in combination with identifying and, optionally, quantitating a fungal mycotoxin in a patient tissue or a patient body fluid. In this embodiment, the mycotoxin can be identified by a method comprising the steps of extracting and recovering the mycotoxin from the patient tissue or body fluid, contacting the mycotoxin with an antibody directed against the mycotoxin, and identifying the myocotoxin.

Illustratively, patient (e.g., human or animal) tissue is received in 1.) a 10% formalin fluid or 2.) in a paraffin block in which the tissue has been fixed in formalin. In one embodiment for mycotoxin detection and quantitation, the tissue can then be processed by various dehydration steps and finally embedded in paraffin. In this embodiment, the tissue can then be cut in 3-5 micron samples. In an illustrative embodiment, approximately 25-35 mg of tissue can then be processed as described in U.S. Appl. Publication No. 20130183697, incorporated herein by reference. Illustratively, body fluids can be prepared as described in U.S. Appl. Publication No. 20130183697, incorporated herein by reference, or by other methods known in the art. In another illustrative embodiment, patient body fluids can be tested for the presence of mycotoxins. Illustratively, any antigen associated with a fungus or with a mycotoxin can be detected. Any of the methods described herein for fungal DNA or mycotoxin identification or quantification or for sample preparation can be preformed as described U.S. Appl. Publication No. 20130183697, incorporated herein by reference.

In one aspect, the methods and compositions for detection and quantification of mycotoxins can also be very specific and sensitive. (e.g., the methods can detect 1.0 ng/ml of aflatoxins, 0.2 ng/ml of tricothecenes, and 2 ng/ml of ochratoxins, or less). Specificity of mycotoxins was tested in each group (Tricothecenes, Aflatoxins, Ochratoxins) by testing known samples of mycotoxins (obtained from Trilogy Laboratories, Washington, Missouri, and from Sigma, St. Louis, Missouri) in each mycotoxin test as described in U.S. Appl. Publication No. 20130183697, incorporated herein by reference. In one embodiment, there were no cross-over reactions or cross-over detection of mycotoxins between the groups. In illustrative embodiments, Enzyme Linked Immunosorbant Assay (ELISA), or affinity chromatography can be used to detect mycotoxins produced by toxic molds. Illustratively, the mycotoxins can be aflatoxins, ochratoxins, or tricothecenes (e.g., Verrucarins A, B and J, Roridin A, E, H, and L-2, Satratoxins F, G, and H, Verrucarol, isosatratoxin F, G, and H, and T-2, or other macrocyclic mycotoxins). Illustrative of antibody-based assays that can be used to identify mycotoxins are the Tricothecene kit (Envirologix, Inc., Portland, Maine), the AFLATEST® (VICAM, Inc.), the OCHRATEST™ (VICAM Inc.), and LUMINEX®-based assays.

In the embodiment where mycotoxins are also identified and quantitated, control samples of the patient body fluid or patient tissue sample to be analyzed can be obtained from patients with no documented history of exposure to molds or mycotoxins. For example, negative control samples can be obtained from autopsy specimens for which the patient had no exposure to mycotoxins or molds (e.g., victims of motor vehicle accidents, coronary artery disease, or myocardial infarction). For positive controls, for example, samples of negative tissue and/or body fluids can be spiked with known positive amounts of mycotoxins or spores prior to evaluation to generate a calibration curve. In another aspect, a calibration reagent (or multiple calibration reagents) can also be used to identify and quantitate mycotoxins. A "calibration reagent" means any standard or reference material containing a known amount of the mycotoxin (i.e., a mycotoxin or a mycotoxin antigen). In this embodiment, the sample suspected of containing the mycotoxin and the calibration reagent (or multiple calibration reagents) can be assayed under similar conditions, and the mycotoxin concentration is then calculated by comparing the results obtained for the unknown sample with the results obtained for the calibration reagent(s). Illustrative calibrators for the mycotoxins can be obtained from producers of the Tricothecene kit (Envirologix, Inc., Portland, Maine) and producers of the AFLATEST® and OCHRATEST™ kits (VICAM Inc., Watertown, Massachusetts), or from Trilogy Laboratories (Washington, Missouri). In another embodiment, Beacon Analytical Systems Inc. kits (Saco, Maine) can be used for the detection of mycotoxins.

In one embodiment, an appropriate instrument for PCR can be used to amplify or detect an amplicon or to quantitate fungal DNA in the two-step PCR method as described herein (e.g. APPLIED BIOSYSTEMS® 7500 Fast instrument or CEPHEID SMART CYCLER® II Instrument). In another embodiment, the first amplification step may be performed using a SMART CYCLER® II instrument. In another embodiment, the second amplification or detection step may be performed using an APPLIED BIOSYSTEMS® 7500 Fast instrument. In another aspect, the DNA amplified is fungal DNA. In another aspect, the DNA detected or quantified is fungal DNA. In these embodiments, any combination of instruments can be used in the first and second amplification and detection steps, and/or for quantitation.

In another embodiment, a method is provided for determining if a patient is at risk for or has developed a disease state related to a fungal infection. The method comprises extracting and recovering fungal DNA of the fungal species from the patient tissue or the patient body fluid, amplifying the fungal DNA using a first set of primers to produce an amplicon, amplifying the amplicon using a second set of primers, hybridizing an isolated probe to the amplicon to identify the specific fungal species, wherein the probe is labeled with at least one fluorescent dye, identifying the specific fungal species to determine if the patient is at risk for or has developed the disease state related to a fungal infection.

In any embodiment involving "determining if the patient has developed the disease state related to the fungal infection," this phrase means "diagnosing the patient to determine if the patient has a fungal infection."

Thus, these method embodiments provide methods of diagnosing fungal infections. Patients in need of diagnosis of a fungal infection can include cancer patients, post-operative patients, transplant patients, patients undergoing chemotherapy, immunosuppressed patients, and the like. These patients may experience symptoms of fungal infections including sinusitis, allergic reactions, headaches, and skin rashes. In one embodiment, patients in need of diagnosis may include humans or animals.

In one embodiment, for diagnosing fungal infections, kits are provided. The kits are useful for identifying, detecting, or quantitating microbial DNA (e.g., fungal DNA) in a patient tissue or body fluid. In one embodiment, a kit is provided comprising a purified nucleic acid with a sequence selected from SEQ ID NOS: 1 to 18, 55, and 56, or with a complement of a sequence selected from SEQ ID NOS: 1 to 18, 55, and 56, and a fluorescently labeled probe. In the embodiment where the kit is used to identify fungal DNA, the kit can contain the probes and/or primers described herein, such as a sequence selected from SEQ ID NOS: 1 to 18, 55, and 56 or a complement of a sequence selected from SEQ ID NOS: 1 to 18, 55, and 56, components to extract and isolate fungal DNA, and components for DNA amplification, such as a heat stable DNA polymerase (e.g., Taq polymerase or Vent polymerase), buffers, MgCl₂, H₂O, and the like. In one embodiment, the reagents can remain in liquid form. In another embodiment, the reagents can be lyophilized. In another illustrative embodiment, the kit can also contain instructions for use.

In another embodiment, a kit is provided comprising a purified nucleic acid with a sequence selected from SEQ ID NOS: 1 and 2, SEQ ID NOS: 3 and 4, SEQ ID NOS: 5 and 6, SEQ ID NOS: 7 and 8, SEQ ID NOS: 9 and 10, SEQ ID NOS: 11 and 12, SEQ ID NOS: 13 and 14, SEQ ID NOS: 15 and 16, SEQ ID NOS: 17 and 18, SEQ ID NOS: 55 and 56, and SEQ ID NOS: 60 and 61, or with a complement of a sequence selected from SEQ ID NOS: 1 and 2, SEQ ID NOS: 3 and 4, SEQ ID NOS: 5 and 6, SEQ ID NOS: 7 and 8, SEQ ID NOS: 9 and 10, SEQ ID NOS: 11 and 12, SEQ ID NOS: 13 and 14, SEQ ID NOS: 15 and 16, SEQ ID NOS: 17 and 18, SEQ ID NOS: 55 and 56, and SEQ ID NOS: 60 and 61, and a fluorescently labeled probe.

In yet another embodiment, a kit is provided comprising a purified nucleic acid with a sequence selected from SEQ ID NOS: 20 and 21, SEQ ID NOS: 23 and 24, SEQ ID NOS: 26 and 27, SEQ ID NOS: 29 and 30, SEQ ID NOS: 32 and 33, SEQ ID NOS: 35 and 36, SEQ ID NOS: 38 and 39, SEQ ID NOS: 41 and 42, SEQ ID NOS: 44 and 45, SEQ ID NOS: 47 and 48, SEQ ID NOS: 58 and 59, and SEQ ID NOS: 63 and 64, or with a complement of a sequence selected from SEQ ID NOS: 20 and 21, SEQ ID NOS: 23 and 24, SEQ ID NOS: 26 and 27, SEQ ID NOS: 29 and 30, SEQ ID NOS: 32 and 33, SEQ ID NOS: 35 and 36, SEQ ID NOS: 38 and 39, SEQ ID NOS: 41 and 42, SEQ ID NOS: 44 and 45, SEQ ID NOS: 47 and 48, SEQ ID NOS: 58 and 59, and SEQ ID NOS: 63 and 64, and a fluorescently labeled probe.

In one embodiment, the fluorescently labeled probe comprises a sequence selected from the group consisting of SEQ ID NOS: 19, 22, 25, 28, 31, 34, 37, 40, 43, 46, and 57. In yet another embodiment, the fluorescently labeled probe comprises a sequence selected from the group consisting of SEQ ID NOS: 19, 22, 25, 28, 31, 34, 37, 40, 43, 46, 57, and 62.

In another embodiment, a purified or isolated nucleic acid is provided with a sequence selected from SEQ ID NOS: 1 to 18, 55, and 56 in combination with fluorescently labeled target DNA. In still another embodiment, a purified or isolated nucleic acid that hybridizes under highly stringent conditions to a sequence selected from SEQ ID NOS: 1 to 18, 55, and 56 is provided in combination with fluorescently labeled target DNA. In yet another embodiment, a purified or isolated nucleic acid is provided comprising a complement of a sequence selected from SEQ ID NOS: 1 to 18, 55, and 56 in combination with fluorescently labeled target DNA. In another embodiment, a purified or isolated nucleic acid is provided with a sequence selected from SEQ ID NOS: 1 to 18, 55, 56, 60, and 61, or complements thereof, in combination with fluorescently labeled target DNA.

In accordance with the methods and compositions described herein, "highly stringent conditions" means hybridization at 65 °C in 5X SSPE and 50% formamide, and washing at 65 °C in 0.5X SSPE.

The following examples provide illustrative methods for carrying out the practice of the present invention. As such, these examples are provided for illustrative purposes only and are not intended to be limiting. As used herein Double Amplification Real-time Polymerase Chain Reaction (DART PCR) refers to an embodiment of the two-step PCR method as described in the EXAMPLES.

### EXAMPLE 1

### SAMPLES AND SAMPLE PREPARATION

Human urine was received in 5-10 mL quantities as first in the morning voided urines. Serums were received with the blood clot removed prior to receipt and a minimum of 1 mL of serum was frozen or used. Nasal secretions were obtained from hospital patients or out-patients. Fixed autopsy and surgical biopsy specimens were obtained from patients who had a history of exposure to fungi. These samples were obtained from hospital pathology departments or coroners' offices. Tissue samples and body fluid samples were also obtained from patients who had no exposure to fungi and were used as a negative control group.

### EXAMPLE 2

### BLOOD NUCLEIC ACID EXTRACTION

Preparation and the extraction of total nucleic acid from whole blood, blood card, plasma, serum, buffy coat, lymphocytes, and body fluids was accomplished using the following procedure.

### Specimens

Whole blood, blood card, plasma, serum, buffy coat, lymphocytes, and body fluids were used for this extraction protocol. 200.0 uL of sample was used for this extraction. After collection red blood cells are stored at 4°C and blood cards may be stored at room temperature until processed.

### Materials

QIAAMP® DSP DNA Mini Kit (Cat. No. 51306; obtained from Qiagen Inc.) for purification of DNA, QIAAMP® DSP DNA Mini Kit mini spin columns in 2 mL collection tubes, 2 mL collection tubes, Buffer ATL, Buffer AL, Buffer AW1, Buffer AW2, Buffer AE (buffers from, for example, QIAAMP® DSP DNA Mini Kit, cat. no. 61304 Qiagen Inc.), Proteinase K, Ethanol (96-100%), 1.5 mL and 2.0 mL microcentrifuge tubes, 15.0 mL conical tubes, Rnase-free, sterile pipet tips with aerosol barrier for pipettes, blood cards when appropriate, and Phosphate Buffered Solution (PBS) were used. The buffers and Proteinase K (PK) were all stored at room temperature and the spin columns were stored at 2-8°C.

### Procedure

Blood Card Preparation (If needed). A blood card was inoculated with four drops of whole blood provided by a patient or control making sure to totally cover the inoculation square of the blood card and allowed to dry completely. In other embodiments, the blood card is prepared by a third party and provided. Next, a sterile scalpel was used to cut out the dried blood square from the blood card and placed in a sterile 15.0 mL conical tube. Then, 500.0 uL of PBS was added to the 15.0 mL conical tube containing the blood card square. The solution was set for 1 hour with periodic vortexing of the solution. The conical tubes were stored at 4°C until ready for processing.

Whole Blood Preparation (if needed). Whole blood contained in a tube provided by a patient or control was spun in a centrifuge at 20,000 RPM for ten minutes. Red and white cells at the bottom of the tube were pipetted out and moved into a labeled 2.0 mL microcentrifuge tube. The cells were stored at 4°C until ready for further processing.

Buffers. Buffers ATL and AL may form precipitates upon storage. If a precipitate formed in either buffer, the buffer was incubated at 55°C until the precipitate had fully dissolved. Buffers AW1 and AW2 were supplied as concentrates. Before using these buffers for the first time, the appropriate amounts of ethanol (96-100%) were added to Buffers AW1 and AW2 as indicated on the bottles. A heat block was set to 55°C for use in the procedure.

Preparation of Samples. 2.0 mL tubes, spin columns, and final collection tubes were labeled with the number corresponding to the patient sample or control. Samples were equilibrated to room temperature if refrigerated or frozen prior to beginning the procedure. In order to extract nucleic acids from the sample, 20.0 uL of PK was pipetted into the bottom of each 2.0 mL microcentrifuge tube. 200.0 uL of sample was added to the microcentrifuge tube containing the PK. Next, 200.0 uL Buffer ALwas added to the sample, and mixed by pulse-vortexing for fifteen seconds. The sample was then incubated at 56°C for ten minutes. The 2.0 mL microcentrifuge tube was briefly centrifuged to remove drops from the inside of the lid. Next, 200.0uL ethanol (96-100%) was added to the sample and mixed again by pulse-vortexing for fifteen seconds. After mixing, the 2.0 mL microcentrifuge tube was briefly centrifuged to remove drops from inside the lid.

Next, the liquid mixture was pipetted from each 2.0 mL microcentrifuge tube and moved into the corresponding mini spin column that sat in a 2 mL collection tube. The columns were centrifuged in a microcentrifuge at 8000 RPM for one minute, and the collection tubes containing flow through were discarded. Each spin column was placed in a new 2.0 mL collection tube. 500.0 uL of Buffer AW1 was added to each column, centrifuged at 8000 RPM for one minute, and the collection tubes containing flow through were discarded. Each spin column was placed in a new 2.0 mL collection tube. 500.0 uL of Buffer AW2 was added to each column and centrifuged at 13,000 RPM for five minutes. Each spin column was placed in a new 2.0 mL collection tube and centrifuge at 13,000 RPM for one minute. Spin columns were removed carefully from the collection tubes and collection tubes containing flow through were discarded. Spin columns were placed in their corresponding 1.5 mL elution tube. 200.0 uL of Buffer AE was pipetted into each spin column and incubated for one minute at room temperature. The spin columns were centrifuged at 8000 RPM for one minute, the spin columns and caps were discarded, and the extracted nucleic acid samples were stored at -10 to -25.9°C.

### EXAMPLE 3

### FIRST AMPLIFICATION METHOD USING REAL-TIME PCR OF EXTRACTED NUCLEIC ACIDS FROM A BLOOD SAMPLE

A procedure for an initial amplification step in the Double Amplification Real-time Polymerase Chain Reaction (DART PCR) methodology described herein for the qualitative detection of the following fungal targets using an APPLIED BIOSYSTEMS^{®} 7500 Fast Real-Time PCR platform and SYBR^{®} Green was accomplished using the following procedure.

| **Aspergillus** | **Candida** |
|---|---|
| niger | albicans |
| flavus | kruseii |
| fumigatus | glabrata |
| terreus | tropicalis |

### Specimens

Nucleic acids extracted from whole blood, blood card, plasma, serum, buffy coat, lymphocytes, and body fluids are appropriate specimens for this procedure. A minimum of 200.0 uL of sample was used for the extraction as described in Example 1.

### Materials

DSP Reagents and Consumables. RT²™ SYBR^{®} Green ROX^{®} FAST Mastermix (2) (Cat. No. 330620; obtained from Qiagen Inc.), PCR grade purified water, 96 well real-time optical plates (Cat. No. 4346906; obtained from Life Technologies, Inc.), MICROAMP^{®} optical adhesive film (Cat. No. 4360954 obtained from Life Technologies, Inc.), 1.5 mL tubes, barrier pipette tips capable of 200.0 µL volumes, barrier pipette tips capable of 1000.0 uL volumes, barrier pipette tips capable of 20.0 uL volumes, and specifically engineered first set of primers detailed below were used. Each first set of primers was designed for a specific fungal target. Each first set of primers includes a forward and a reverse primer. SYBR® Green has an absorption wavelength at 494 nm and an emission wavelength at 521 nm.

### Target and corresponding engineered first set of primers:

### Target - Aspergillus niger

Primer NF1 5'-AGGAAGTAAAAGTCGTAACAAG (SEQ ID NO: 1)
Primer NR1 5'-CGCATTTCGCTGCGTTCTTC (SEQ ID NO: 2)

### Target - Aspergillus flavus

Primer NF1 5'-AGGAAGTAAAAGTCGTAACAAG (SEQ ID NO: 3)
Primer NR1 5'-CGCATTTCGCTGCGTTCTTC (SEQ ID NO: 4)

### Target - Aspergillus fumigatus

Primer NF1 5'-AGGAAGTAAAAGTCGTAACAAG (SEQ ID NO: 5)
Primer NR1 5'-CGCATTTCGCTGCGTTCTTC (SEQ ID NO: 6)

### Target - Aspergillus terreus

Primer NATF 5'-GACTATTGTACCTTGTTGCTTC (SEQ ID NO: 7)
Primer NATR 5'-CATTAGTTATCGCATTTCGCTG (SEQ ID NO: 8)

### Target - Candida albicans

Primer NFCA 5'- TAGCGAACAAGTACAGTGATG (SEQ ID NO: 9)
Primer NRCA 5'-CTCGGTCTAGGCTGGCAG (SEQ ID NO: 10)

### Target - Candida tropicalis

Primer NFCT 5'-ATGGAAAGATGAAAAGAACTTTG (SEQ ID NO: 11)
Primer NRCT 5'-GCTGGCAGTATCGACGAAG (SEQ ID NO: 12)

### Target - Candida glabrata

Primer NFCG 5'-GCTTGGGACTCTCGCAG (SEQ ID NO: 13)
Primer NRCG 5'-GGCATATAACCATTATGCCAG (SEQ ID NO: 14)

### Target - Candida krusei

Primer NFCK 5'-AAACCAACAGGGATTG (SEQ ID NO: 15)
Primer NRCK 5'-CCCAAACAACTCGAC (SEQ ID NO: 16)

### Procedure

Processing Controls. Every clinical sample processed was inoculated with spores from the internal control target *Geometrica* to show that a negative target result is a true negative result and not related to the extraction of the sample. The samples were processed, amplified, and detected utilizing primer and probes specific for *Geometrica.* A positive control for each target of interest was processed along with each clinical sample in each real-time PCR run. This positive control was extracted from tissue, spore solutions, or purchased from a vendor. The positive control showed that the primer and probe set for each target (as described in Example 2 and Example 5) is not being inhibited and showed that a negative result is a true negative. A negative control for each target of interest was processed along with each clinical sample in each real-time PCR run. The negative control was extracted from tissue or water. The negative control showed that the primer and probe set (as described in Example 2 and Example 5), water, and extraction reagents for each target were not contaminated with the target and showed that a positive result is a true positive. The controls were tested in parallel with kit lots currently in use.

Dilution of Primer Stocks. Primers were stored at -10 to -25.9°C and upon reconstitution were stored at room temperature while stil lyophilized. Lyophilized primers were resuspended in PCR grade water to a final concentration of 100 uM. As an illustration, if the synthesis yields 38.6 nMoles, then 386 uL of PCR grade water was added to achieve 100uM concentration. A 10 uM working stock from the 100 uM primer stocks was prepared by adding 50 uL of 100 uM stock primer to 450 uL of molecular grade water for a final volume of 500 uL and a final concentration of 10 uM.

Reaction Setup. The reaction setup for one reaction is shown below. A reaction consisted of 19.0 uL of reaction mix and 1.0 uL of extracted nucleic acids (e.g. DNA), and each reaction was run in duplicate or triplicate.

| | Stock Conc. | Work Conc. | Per/RXN |
|---|---|---|---|
| 2x Master Mix | 2X | 1X | 10.0 uL |
| Primer 1 | 10 uM | .5 uM | 1.0 uL |
| Primer 2 | 10 uM | .5 uM | 1.0 uL |
| Water | na | na | 7.0 uL |
| | | TOTAL | 19.0 uL |

Cycling Parameters. The APPLIED BIOSYSTEMS^{®} 7500 Fast instrument and software cycling profile is illustrated for the nested procedure and the run parameters for this program are outlined below.

### Step 1 (1 Cycle)

Hot Start: 95°C for 10 minutes

### Step 2 (40 cycles)

Denature: 95°C for 10 seconds
Anneal: 60°C 30 seconds

### Master Mix Preparation.

A 1.5 mL tube was labeled for each target requiring a master mix and placed in a tube rack inside a PCR cabinet containing thawed working primer and primer/probe stocks. Approximately 1.0 mL of PCR grade purified water to be used in the master mix setup was aliquoted into a 1.5 mL tube. Next, the appropriate amount of RT²™ SYBR^{®} Green ROX^{®} FAST Mastermix (2) was pipetted into each of the labeled 1.5 mL reaction tubes. Next, the appropriate amount of each primer working stock was pipetted into each master mix tube. In this step two primers, one forward and one reverse, were added to the assay. Next, the appropriate amount of molecular grade water was added to each master mix tube. After all components have been delivered into the appropriate 1.5 mL tubes, the tubes were capped and solutions mixed completely utilizing a tube vortexer. Volumes may differ depending on the number of reactions processed. An example reaction mix is as follows:

| ***Assay 1*** | **7** | **RXN #** | **12** | **Plus (10%)** | **13.0** |
|---|---|---|---|---|---|
| ***20.0 uL Reaction with 19.0 uL of mastermix and 1.0 uL of DNA*** | | | | | |
| | **Stock Conc.** | **Work Conc.** | **Per/RXN** | **Mastermix** | |
| ***2x mm*** | 2X | 1X | 10.0 | **130.0** | |
| ***PRI R*** | 10 uM | .5 uM | 1.0 | **13.0** | |
| ***PRI F*** | 10 uM | .5 uM | 1.0 | **13.0** | |
| ***Water*** | na | Na | 7.0 | **91.0** | |

Mini Mix Preparation and Plate Loading. Each 1.5 mL tubes for each sample, the positive control, the negative control, and the no template control were placed in a 96 well tube rack. 60.8 uL (for triplicate) or 40.8 uL (for duplicate) of each master mix was pipetted into the corresponding mini mix tubes. 3.2 uL (for triplicate) or 2.2 uL (for duplicate) of extracted sample DNA or control DNA was pipetted into the tubes. Then all 1.5 mL tubes were closed and vortexed. In a 96 well real-time optical plate, 20.0 uL of each mini mix containing sample or control was pipetted into three wells (for triplicate) or two wells (for duplicate) of an optical plate. After the optical plate was loaded, the plate was lightly tapped on the bench top several times to insure that the liquid was at the bottom of the well. Optical adhesive film was applied to the plate evenly and covered all 96 wells of the optical plate. The plate was placed in the 4°C refrigerator while the run setup on the APPLIED BIOSYSTEMS^{®} 7500 Fast instrument was performed.

APPLIED BIOSYSTEMS^{®} 7500 Fast Setup and Run. The APPLIED BIOSYSTEMS^{®} 7500 Fast instrument and software was used to analyze the amplification.

Results Interpretation. A positive result was defined as any amplification observed crossing the fluorescence baseline threshold between cycles 1 and 40 of the real-time PCR run. A negative result was defined as no amplification observed crossing the fluorescence baseline threshold between cycles 1 and 40 of the PCR run. An equivocal result was defined as amplification observed crossing the fluorescence baseline threshold after or at cycle 40, a control out of range, or questions regarding sample integrity. A control that was positive for the target being tested and showed that the assay detected the presence of target DNA and that there was not PCR inhibition was a valid positive control. A control that was negative for the target being tested and showed that the reagents or the sample were not contaminated with the target prior to the testing of the sample was a valid negative control.

The following are exemplary tables:

| **Reportable Result** | **Crossing Poin**t | **Positive Control** | **Negative Control** |
|---|---|---|---|
| Positive Result | <40 | (+) | (-) |
| Positive Result | <40 | (-) | (-) |
| Positive Result | <40 | (+) | (-) |
| Positive Result | <40 | (-) | (-) |
| Negative Result | (-) | (+) | (-) |
| Negative Result | (-) | (+) | (+) |
| Negative Result | (-) | (-) | (+) |

| **Un-reportable Result** | **Crossing Point** | **Positive Control** | **Negative Control** |
|---|---|---|---|
| Positive Result | <40 | (+) | (+) |
| Positive Result | <40 | (-) | (+) |
| Positive Result | <40 | (+) | (+) |
| Positive Result | <40 | (-) | (+) |
| Negative Result | (-) | (-) | (-) |
| Negative Result | (-) | (+) | (-) |
| Negative Result | (-) | (+) | (+) |

### EXAMPLE 4

### ALTERNATIVE FIRST AMPLIFICATION METHOD USING PCR OF EXTRACTED NUCLEIC ACIDS FROM A BLOOD SAMPLE

An alternative procedure for an initial amplification step in the Double Amplification Real-time Polymerase Chain Reaction (DART PCR) methodology for the qualitative detection of fungal targets uses an APPLIED BIOSYSTEMS^{®} 7500 Fast or EPPENDORF® MASTERCYCLER® PCR platform and gel electrophoresis according to the following procedure.

### Specimens

Whole blood, blood card, plasma, serum, buffy coat, lymphocytes, and body fluids are appropriate specimens for this extraction protocol. 200.0 uL samples were used for this extraction. After collection red blood cells were stored a 4°C and blood cards were stored at room temperature until processed.

### Materials

Taq DNA Polymerase 250U (Cat. No. 201203; obtained from Qiagen Inc.; stored at -20°C), dNTP Mixture 25mM (Cat. No. 201203; obtained from New England Biolabs), BSA Solution 8ug/uL (stored at 4°C), PCR grade purified water, eight well strip tubes (Cat. No. 10-177; obtained from Genesee Scientific), barrier pipette tips capable of 200.0 µL volumes, barrier pipette tips capable of 1000.0 uL volumes, barrier pipette tips capable of 20.0 uL, and specifically engineered first set of primers as detailed in Example 2 were used. Each first set of primers was designed for a specific fungal target. Each first set of primers includes a forward and a reverse primer.

### Procedure

Processing Controls. Controls were processed as previously described in Example 3.

Dilution of Primer Stocks. Primer stocks were diluted as previously described in Example 3.

Reaction Setup. The reaction setup for an example reaction is shown below. A reaction consisted of 20.0 uL of reaction mix and 5.0 uL of extracted nucleic acids (e.g. DNA), and each reaction was run in duplicate or triplicate.

| | Stock Conc. | Per/RXN |
|---|---|---|
| PCR Buffer | 10X | 2.5 uL |
| MgCl2 (As Needed)* | 25mM | NA |
| dNTP | 10mM | 1.0 uL |
| Primer 1 (Ex. NFCA) | 10uM | 2.0 uL |
| Primer 2 (Ex. NRCA) | 10uM | 2.0 uL |
| Enzyme (Taq) | 2.5U | .5 uL |
| ddH2O | NA | 12.0 uL |
| | Total | 20.0 uL |
| Adjuvant | 8ug/uL | 5.0 uL |

MgCl₂ was used as needed in the reaction setup (see further setup examples below). The reaction setup for a master mix was prepared for multiple reactions using the target Specific Worksheet tables below.

Use setup for: A. fumigatus, A. flavus, A. niger

| Controls: 8 | | | | Date: MM/DD/YR | |
|---|---|---|---|---|---|
| Primers: NF1, NR1 | | | | Initials: XXX | |
| DNA Numbers: See Below | | | | Project: Initial Amp DART | |
| | | | | | |
| Reagents | Man./Lot# | Stock Conc. | Working Conc. | Per rxn | Master Mix X 4 |
| PCR buffer | Qiagen/12332 | 10X | | 2.5 uL | 10.0 uL |
| MgCl₂ | Not Used | 25mM | | NA | NA |
| dNTP | NEB/24678 | 10mM | | 1.0 uL | 4.0 uL |
| Primer 1 | NFI/IDT 2468 | 10uM | | 2.0 uL | 8.0 uL |
| Primer 2 | NRI/IDT 3456 | 10uM | | 2.0 uL | 8.0 uL |
| Enzyme | Qiagen/12332 | 5U/uL | | .5 uL | 2.0 uL |
| ddH₂O | VWR/2345 | | | 12.0 uL | 48.0 uL |
| Adjuvant | BSA/041215 | 8ug/uL | | 5.0 uL | 20.0 uL |
| Template | Extracted Plasma | | | 5.0 uL | |
| | | | | T.V.: 25.0 uL | |

Program: 60 Degree - 10 minute Hot Start

Loaded directly - PCR buffer contains loading dye.

| POS | NTC | C2016-35 (Sample) | C2016-36 (Negative) | | | | |
|---|---|---|---|---|---|---|---|
| | | | | | | | |
| | | | | | | | |
| | | | | | | | |

**Use setup for: A. terreus**

| Controls: 25 | | | | Date: MM/DD/YR | |
|---|---|---|---|---|---|
| Primers: ATF, ATR | | | | Initials: XXX | |
| DNA Numbers: See Below | | | | Project: Initial Amp DART | |
| Reagents | Man./Lot# | Stock Conc. | Working Conc. | Per rxn | Master Mix X 4 |
| PCR buffer | Qiagen/12332 | 10X | | 2.5 uL | 10.0 uL |
| MgCl₂ | Not Used | 25mM | | NA | NA |
| dNTP | NEB/24678 | 10mM | | 1.0 uL | 4.0 uL |
| Primer 1 | ATF/IDT 2468 | 10uM | | 2.0 uL | 8.0 uL |
| Primer 2 | ATR/IDT 3456 | 10uM | | 2.0 uL | 8.0 uL |
| Enzyme | Qiagen/12332 | 5U/uL | | .5 uL | 2.0 uL |
| ddH₂O | VWR/2345 | | | 12.0 uL | 48.0 uL |
| Adjuvant | BSA/041215 | 8ug/uL | | 5.0 uL | 20.0 uL |
| Template | Extracted Plasma | | | 5.0 uL | |
| | | | | T.V.: 25.0 uL | |

Program: 60 Degree - 10 minute Hot Start

Loaded directly - PCR buffer contains loading dye.

| POS | NTC | C2016-35 (Sample) | C2016-36 (Negative) | | | | |
|---|---|---|---|---|---|---|---|
| | | | | | | | |
| | | | | | | | |
| | | | | | | | |

Use setup for: C. albicans

| Controls: CA | | | | Date: MM/DD/YR | |
|---|---|---|---|---|---|
| Primers: NFCA, NRCA | | | | Initials: XXX | |
| DNA Numbers: See Below | | | | Project: Initial Amp DART | |
| | | | | | |
| Reagents | Man./Lot# | Stock Conc. | Working Conc. | Per rxn | Master Mix X 4 |
| PCR buffer | Qiagen/12332 | 10X | | 2.5 uL | 10.0 uL |
| MgCl₂ | Not Used | 25mM | | NA | NA |
| dNTP | NEB/24678 | 10mM | | 1.0 uL | 4.0 uL |
| Primer 1 | NFCA/IDT 2468 | | | 2.0 uL | 8.0 uL |
| Primer 2 | NRCA/IDT 3456 | 10uM | | 2.0 uL | 8.0 uL |
| Enzyme | Qiagen/12332 | 5U/uL | | .5 uL | 2.0 uL |
| ddH₂O | VWR/2345 | | | 12.0 uL | 48.0 uL |
| Adjuvant | BSA/041215 | 8ug/uL | | 5.0 uL | 20.0 uL |
| Template | Extracted Plasma | | | 5.0 uL | |
| | | | | T.V.:25.0 uL | |

Program: 60 Degree - 10 minute Hot Start

Loaded directly - PCR buffer contains loading dye.

| POS | NTC | C2016-35 (Sample) | C2016-36 (Negative) | | | | |
|---|---|---|---|---|---|---|---|
| | | | | | | | |
| | | | | | | | |
| | | | | | | | |

Use setup for: C. glabrata

| Controls: CG | | | | Date: MM/DD/YR | |
|---|---|---|---|---|---|
| Primers: NFCG, NRCG | | | | Initials: XXX | |
| DNA Numbers: See Below | | | | Project: Initial Amp DART | |
| | | | | | |
| Reagents | Man./Lot# | Stock Conc. | Working Conc. | Per rxn | Master Mix X 4 |
| PCR buffer | Qiagen/12332 | 10X | | 2.5 uL | 10,0 uL |
| MgCl₂ | Not Used | 25mM | | NA | NA |
| dNTP | NEB/24678 | 10mM | | 1.0 uL | 4.0 uL |
| Primer 1 | NFCG/IDT 2468 | 10uM | | 2.0 uL | 8.0 uL |
| Primer 2 | NRCG/IDT 3456 | 10uM | | 2.0 uL | 8.0 uL |
| Enzyme | Qiagen/12332 | 5U/uL | | .5 uL | 2.0 uL |
| ddH₂O | VWR/2345 | | | 12.0 uL | 48.0 uL |
| Adjuvant | BSA/041215 | 8ug/uL | | 5.0 uL | 20.0 uL |
| Template | Extracted Plasma | | | 5.0 uL | |
| | | | | T.V.:25.0 uL | |

Program: 60 Degree - 10 minute Hot Start

Loaded directly - PCR buffer contains loading dye.

| POS | NTC | C2016-35 (Sample) | C2016-36 (Negative) | | | | |
|---|---|---|---|---|---|---|---|
| | | | | | | | |
| | | | | | | | |
| | | | | | | | |

Use setup for: C. tropicalis

| Controls: CT | | | | Date: MM/DD/YR | |
|---|---|---|---|---|---|
| Primers: NFCT, NRCT | | | | Initials: XXX | |
| DNA Numbers: See Below | | | | Project: Initial Amp DART | |
| | | | | | |
| Reagents | Man./Lot# | Stock Conc. | Working Conc. | Per rxn | Master Mix X 4 |
| PCR buffer | Qiagen/12332 | 10X | | 2.5 uL | 10.0 uL |
| MgCl₂ | Qiagen/12332 | 25mM | | .5 uL | 2.0 uL |
| dNTP | NEB/24678 | 10mM | | 1.0 uL | 4.0 uL |
| Primer 1 | NFCA/IDT 2468 | 10uM | | 2.0 uL | 8.0 uL |
| Primer 2 | NRCA/IDT 3456 | 10uM | | 2.0 uL | 8.0 uL |
| Enzyme | Qiagen/12332 | 5U/uL | | .5 UL | 2.0 uL |
| ddH₂O | VWR/2345 | | | 11.5 uL | 46.0 uL |
| Adjuvant | BSA/041215 | 8ug/uL | | 5.0 uL | 20.0 uL |
| Template | Extracted Plasma | | | 5.0 uL | |
| | | | | T.V.: 25.0 uL | |

Program: 60 Degree - 10 minute Hot Start

Loaded directly - PCR buffer contains loading dye.

| POS | NTC | C2016-35 (Sample) | C2016-36 (Negative) | | | | |
|---|---|---|---|---|---|---|---|
| | | | | | | | |
| | | | | | | | |
| | | | | | | | |

Use setup for: C. kruseii

| Controls: CK | | | | Date: MM/DD/YR | |
|---|---|---|---|---|---|
| Primers: NFCK, NRCK | | | | Initials: XXX | |
| DNA Numbers: See Below | | | | Project: Initial Amp DART | |
| | | | | | |
| Reagents | Man./Lot# | Stock Conc. | Working Conc. | Per rxn | Master Mix X4 |
| PCR buffer | Qiagen/12332 | 10X | | 2.5 uL | 10.0 uL |
| MgCl₂ | Qiagen/12332 | 25mM | | .5 uL | 2.0 uL |
| dNTP | NEB/24678 | 10mM | | 1.0 uL | 4.0 uL |
| Primer 1 | NFCK/IDT 2468 | 10uM | | 2.0 uL | 8.0 uL |
| Primer 2 | NRCK/DT 3456 | 10uM | | 2.0 uL | 8.0 uL |
| Enzyme | Qiagen/12332 | 5U/uL | | | 2.0 uL |
| ddH₂O | VWR/2345 | | | 11.5 uL | 46.0 uL |
| Adjuvant | BSA/041215 | 8ug/uL | | 5.0 uL | 20.0 uL |
| Template | Extracted Plasma | | | 5.0 uL | |
| | | | | T.V.: 25.0 uL | |

Program: 54 Degree - 5 minute Hot Start

Loaded directly - PCR buffer contains loading dye.

| POS | NTC | C2016-35 (Sample) | C2016-36 (Negative) | | | | |
|---|---|---|---|---|---|---|---|
| | | | | | | | |
| | | | | | | | |
| | | | | | | | |
| | | | | | | | |

### Cycling Parameters (Cycling Parameters used for Specific Targets).

*Aspergillus fumigatus, flavus, niger,* and *terreus*
   *Candida albicans, glabrata, tropicalis*

### Step 1 (1 Cycle)

| | |
|---|---|
| Hot Start: | 95°C for 10 minutes |

### Step 2 (35 cycles)

| | |
|---|---|
| Denature: | 94°C for 45 seconds |
| Anneal: | 60°C for 45 seconds |
| Extension: | 72°C for 1 minute |

### Step 3 (1 Cycle)

| | |
|---|---|
| Extension: | 72°C for 7 minutes |
| Hold: | 4°C Indefinitely |

### Candida Kruseii

### Step 1 (1 Cycle)

| | |
|---|---|
| Hot Start: | 95°C for 5 minutes |

### Step 2 (35 cycles)

| | |
|---|---|
| Denature: | 94°C for 45 seconds |
| Anneal: | 54°C for 45 seconds |
| Extension: | 72°C for 1 minute |

### Step 3 (1 Cycle)

| | |
|---|---|
| Extension: | 72°C for 7 minutes |
| Hold: | 4°C Indefinitely |

Master Mix Preparation. A target Specific Worksheet table described above was used to prepare the amount of master mix needed. A 1.5 mL tube for each target requiring a master mix was labeled and placed in a tube rack inside the PCR cabinet also containing the working primer stocks. Approximately 1.0 mL of PCR grade purified water was aliquoted into an additional 1.5 mL tube to be used in the master mix. The appropriate amount of QIAGEN^{®} Buffer, MgCl₂ and water was pipetted into each of the labeled 1.5 mL reaction tubes based on the Specific Worksheet created for the run. Next the appropriate amount of each primer set working stock was pipetted into each master mix tube. After the primers were delivered, the appropriate amount of dNTP and QIAGEN^{®} Taq DNA polymerase were pipetted into each master mix tube. Lastly, the appropriate amount of bovine serum albumin (BSA) adjuvant was pipetted into each master mix tube. After all components were delivered into the appropriate 1.5 mL tubes, the tubes were capped and the solution was mixed completely using a tube vortexer.

Master Mix Aliquoting and Template Addition. Using the Specific Worksheet as a guide, the appropriate number of 8-well strip tubes were placed in a 96-well microtube rack. The tubes and lids were labeled by worksheet row. Using the Specific Worksheet as a guide, 20.0 uL of each master mix was pipetted into the appropriate wells of the 8-well strip tubes. 5.0 uL of template, controls, and water were pipetted into the appropriate wells in the 96-well microtube rack. After the 8-well strip tubes were loaded and capped, the microtube rack containing the 8-well strip tubes was vortexed then lightly tapped on the bench top several times to insure that the liquid was at the bottom of the well. Then the 8-well strip tubes were removed from the microtube rack and placed in the thermal cycler.

Thermal Cycler Setup and Run. The EPPENDORF^{®} MASTERCYCLER^{®} gradient thermal cycler and software were enabled and the assay was run following the proper programing. Once the program finished running the assay, the samples were removed and placed in -20°C freezer or used in the gel electrophoresis step.

Gel Electrophoresis and Picture. The amplicon was taken from the thermal cycler tubes and controls and samples were run on an agarose gel. Upon completion of electrophoresis, a picture of the gel results was taken.

Results Interpretation. A result passed and could move on to the second amplification procedure if the following are observed:
1. Positive Control has a band;
2. Negative Control has no band;
3. No Template Control has no band; and
4. Samples may or may not have a band.

### EXAMPLE 5

### SECOND AMPLIFICATION METHOD USING REAL-TIME PCR OF EXTRACTED NUCLEIC ACIDS FROM A BLOOD SAMPLE

A procedure for a second amplification step in the Double Amplification Real-time Polymerase Chain Reaction (DART PCR) methodology for the detection of the fungal targets described in Example 2 using an APPLIED BIOSYSTEMS^{®} 7500 Fast real-time PCR platform and a second set of engineered primers and probes was accomplished using the following procedure.

### Specimens

An amplicon from the Initial Amplification DART PCR procedure described in Example 3 or a PCR procedure as described in Example 4 was the appropriate template for this procedure. A 200.0 uL sample was used for this procedure.

### Materials

TAQMAN^{®} Fast Universal Mastermix (2X) (Cat. No. 4352042; obtained from Life Technologies), PCR grade purified water, 96-well real-time optical plates (Cat. No. 4346906; obtained from Life Technologies), MICROAMP^{®} optical adhesive film (Cat. No. 4360954; obtained from Life Technologies), 1.5 mL tubes, barrier pipette tips capable of 200.0 µL volumes, barrier pipette tips capable of 1000.0 uL volumes, and barrier pipette tips capable of 20.0 uL volumes were used.
Engineered primers and probes were used for amplification and detection. Probes were synthesized with either the reporter FAM™ attached to the 5' end of the probe or dCAL FLUOR^{®} Orange 560 attached to the 5' end of the probe. FAM™ has an absorption wavelength at 495 nm and an emission wavelength at 516 nm. dCAL FLUOR^{®} Orange 560 has an absorption wavelength at 538 nm and an emission wavelength at 559 - 560 nm. All probes had the quencher BHQ^{®} attached to the 3' end of the probe. Primers and probes were stored at previously described temperatures. Sequences were as follow:

### Target - Aspergillus niger

Probe niger: 5'-TGTCTATTGTACCCTGTTGCTTC (SEQ ID NO: 19)
Primer F1: 5'-CGTAGGTGAACCTGCGGAAG (SEQ ID NO: 20)
Primer R1: 5'-ATCGATGCCGGAACCAAGAG (SEQ ID NO: 21)

### Target - Geometrica candidum

Probe geo: 5'-AACGCACATTGCACTTTGGGGTATC (SEQ ID NO: 22)
Geo F1H: 5'-GGATCTCTTGGTTCTCGTATC (SEQ ID NO: 23)
Geo R1H: 5'-CTTGATCTGAGGTTGAATAGTG (SEQ ID NO: 24)

### Target - Aspergillus flavus

Probe flav: 5'-CCCGCCATTCATGGCCGCCGGG (SEQ ID NO: 25)
Primer F1: 5'-CGTAGGTGAACCTGCGGAAG (SEQ ID NO: 26)
Primer R1: 5'-ATCGATGCCGGAACCAAGAG (SEQ ID NO: 27)

### Target - Aspergillus fumigatus

Probe fumi: 5'-AAAGTATGCAGTCTGAGTTGATTATC (SEQ ID NO: 28)
Primer F1: 5'-GTAGGTGAACCTGCGGAAG (SEQ ID NO: 29)
Primer R1: 5'- ATCGATGCCGGAACCAAGAG (SEQ ID NO: 30)

### Target - Aspergillus terreus

Probe : 5'-AGTCTGAGTGTGATTCTTTGCAATC (SEQ ID NO: 31)
Primer F: 5'-ACATGAACCCTGTTCTGAAAG (SEQ ID NO: 32)
Primer R: 5'-CCAAGAGATCCATTGTTGAAAG (SEQ ID NO: 33)

### Target - Candida albicans

Probe CA: 5'-TCGGGGGCGGCCGCTGCGG (SEQ ID NO: 34)
Primer F: CA 5'-AAAAAGTACGTGAAATTGTTG (SEQ ID NO: 35)
Primer R: CA 5'-AAGCCGTGCCACATTC (SEQ ID NO: 36)

### Target - Candida krusei

Probe CK: 5'-AAGGCGGTGTCCAAGTCCCTTG (SEQ ID NO: 37)
Primer F: CK 5'-TCAGTAGCGGCGAGTGAAG (SEQ ID NO: 38)
Primer R: CK 5'-AGAAGGGCCTCACTGCTTC (SEQ ID NO: 39)

### Target - Candida glabrata

Probe CG: 5'-ACCTAGGGAATGTGGCTCTGCG (SEQ ID NO: 40)
Primer F: CG 5'-TGGGCCAGCATCGGTTTTG (SEQ ID NO: 41)
Primer R: CG 5' -CCTAGATAACAAGTATCGCAG (SEQ ID NO: 42)

### Target - Candida tropicalis

Probe CT: 5'-TCGGGGGTGGCCTCTACAG (SEQ ID NO: 43)
Primer F: CT 5'-AAAAAGTACGTGAAATTGTTG (SEQ ID NO: 44)
Primer R: CT 5'-AAGCCGTGCCACATTC (SEQ ID NO: 45)

### Procedure

Processing Controls. Controls were processed as previously described in Example 3.

Dilution of Primer Stocks. Primer stocks were diluted as previously described in Example 3.

Dilution of Probe Stocks. The lyophilized probes were resuspended in PCR grade water to a final concentration of 100 uM. For example, if the synthesis yields 15.03 nMoles, 150.3 uL of PCR grade water was added to achieve 100 uM concentration. A 2.5 uM working stock was prepared from the 100 uM probe stocks by adding 12.5 uL of 100 uM stock primer to 487.5 uL of molecular grade water for a final volume of 500 uL and a final concentration of 2.5 uM.

Reaction Setup. The reaction consisted of 19.0 uL of reaction mix and 1.0 uL of DNA and each reaction was run in duplicate or triplicate. The following table illustrates the reaction setup:

| | Stock Conc. | Work Conc. | Per/RXN |
|---|---|---|---|
| 2x Master Mix | 2X | 1X | 10.0 uL |
| Primer 1 | 10 uM | .5 uM | 1.0 uL |
| Primer 2 | 10 uM | .5 uM | 1.0 uL |
| Probe | 2.5 uM | .1 uM | 0.8 uL |
| Water | na | na | 6.2 uL |
| | | TOTAL | 19.0 uL |

Cycling Parameters. The APPLIED BIOSYSTEMS^{®} 7500 instrument and software cycling profile program was used to run the assay. The following was the cycling parameters:

### Step 1 (1 Cycle)

| | |
|---|---|
| Hot Start: | 95°C for 20 seconds |

### Step 2 (40 cycles)

| | |
|---|---|
| Denature: | 95°C for 3 seconds |
| Anneal: | 60°C 30 seconds |

Master Mix Preparation. A 1.5 mL tube for master mix was labeled for each target and placed in a tube rack inside the PCR cabinet containing the working primer/probe stocks. An additional 1.5 mL tube was taken and approximately 1.0 mL of PCR grade purified water was aliquoted into the tube to be used in the master mix setup and the tube placed in the rack. An appropriate amount of the TAQMAN^{®} Fast Universal Mastermix (2X) was pipetted into each of the labeled 1.5 mL reaction tubes based on the PCR worksheet created for the run. After the TAQMAN^{®} Fast Universal Mastermix (2X) is pipetted into the master mix tubes, the appropriate amount of each primer working stock and each probe working stock was added to each master mix tube based on the PCR worksheet created for the run. For each assay, two primers and one probe were used. After the primers and probes have been delivered, the appropriate amount of molecular grade water was added to each master mix tube based on the PCR worksheet created for the run. After all components were delivered into the appropriate 1.5 mL tubes, the tubes were capped and the solution was mixed completely using a tube vortexer. As an example, the following worksheet was used to calculate volumes used for an assay in triplicate:

| | 1 | 2 | 3 | 4 | 5 | 6 | 7 | 8 | 9 | 10 | 11 | 12 |
|---|---|---|---|---|---|---|---|---|---|---|---|---|
| *A* | 7-2012-63 | 7-2012-63 | 7-2012-63 | 7-Neg | 7-Neg | 7-Neg | 7-Pos | 7-Pos | 7-Pos | 7-NTC | 7-NTC | 7-NTC |
| *B* | 7-Initial NTC | 7-Initial NTC | 7-Initial NTC | | | | | | | | | |
| *C* | | | | | | | | | | | | |
| *D* | | | | | | | | | | | | |
| *E* | | | | | | | | | | | | |
| *F* | | | | | | | | | | | | |
| *G* | | | | | | | | | | | | |
| *H* | | | | | | | | | | | | |

| | | | | | |
|---|---|---|---|---|---|
| *Assay 1* | 7 | RXN # | 15 | Plus (10%) | 16.0 |

| 20.0 uL Reaction with 19.0 uL mastermix and 1.0 uL of DNA | | | | | |
|---|---|---|---|---|---|
| | Man/Lot | Stock Conc. | Work Conc. | Per/RXN | Master mix |
| *2x mm* | 1203330 | 2X | 1X | 10.0 | 160.0 |
| *PRI R* | 1016757882 | 10 uM | .5 uM | 1.0 | 16.0 |
| *PRI F* | 1016757882 | 10 uM | .5 uM | 1.0 | 16.0 |
| *PROBE* | 1016757880 | 2.5 uM | .1 uM | 0.8 | 12.8 |
| *Water* | 112102 | na | na | 6.2 | 99.2 |

| Contro ls | Lot |
|---|---|
| | |
| POS | |
| NTC | NA |

| | | |
|---|---|---|
| Run | | 83012.1 |
| Run Date | | MM/DD/YR |
| Techni cian | | XXX |

As another example, the following worksheet was used to calculate volumes used for an assay in duplicate:

| | **1** | **2** | **3** | **4** | **5** | **6** | **7** | **8** | **9** | **10** | **11** | **12** |
|---|---|---|---|---|---|---|---|---|---|---|---|---|
| ***A*** | 7-2012-63 | 7-2012-63 | 7-Neg | 7-Neg | 7-Pos | 7-Pos | 7-NTC | 7-NTC | 7-Initial NTC | 7-Initial NTC | | |
| ***B*** | | | | | | | | | | | | |
| ***C*** | | | | | | | | | | | | |
| ***D*** | | | | | | | | | | | | |
| ***E*** | | | | | | | | | | | | |
| ***F*** | | | | | | | | | | | | |
| ***G*** | | | | | | | | | | | | |
| ***H*** | | | | | | | | | | | | |

| *Assay 1* | 7 | RXN # | 10 | Plus (10%) | 11.0 |
|---|---|---|---|---|---|
| ***20.0 uL Reaction with 19.0 uL of mastermix and 1.0 uL of DNA*** | | | | | |
| | **Mun/Lot** | **Stock Conc.** | **Work Conc.** | **Per/RXN** | **Mastermix** |
| ***2x mm*** | 1203330 | 2X | 1X | 10.0 | 110.0 |
| ***PRI R*** | 101675788 2 | 10 uM | .5 uM | 1.0 | 11.0 |
| ***PRI F*** | 1016757882 | 10 uM | .5 uM | 1.0 | 11.0 |
| ***PROBE*** | 101675788 0 | 2.5 uM | .1 uM | 0.8 | 8.8 |
| ***Water*** | 112102 | na | na | 6.2 | 68.2 |

| Controls | Lot |
|---|---|
| | |
| POS | NA1711 3B2 |
| NTC | NA |

| | | |
|---|---|---|
| Run | | 83012.1 |
| Run Date | | MM/D D/YR |
| Technician | | XXX |

Mini Mix Preparation and Plate Loading. Labeled 1.5 mL tubes for each sample, the positive control, the negative control, and the no template control were placed in a 96-well tube rack. 60.8uL (for triplicate) 40.8uL (for duplicate) of each master mix was pipetted into the corresponding mini mix tubes. Using the worksheet as a guide, 3.5uL (for triplicate) or 2.5uL (for duplicate) of amplicon DNA or control DNA was added to each of the mini mix tubes containing master mix, and then each 1.5 mL tube was vortexed. In a 96-well real-time optical plate, 20.0 uL of each mini mix containing sample or control was pipetted into three wells of the optical plate. After the optical plate is loaded, the plate was lightly tapped on the bench top several times to insure that the liquid was at the bottom of the well. The optical adhesive film was applied to the plate evenly and covered all 96 wells of the optical plate. The plate was placed in the 4°C refrigerator while the APPLIED BIOSYSTEMS® 7500 Fast instrument was setup.

APPLIED BIOSYSTEMS® 7500 Fast Setup and Run. The APPLIED BIOSYSTEMS® 7500 Fast instrument and software was used to perform the assay for the specific FRET probe.

Results Interpretation. A positive result was defined as any amplification observed crossing the fluorescence baseline threshold between cycles 1 and 40 of the real-time PCR run. A negative result was defined as no amplification observed crossing the fluorescence baseline threshold between cycles 1 and 40 of the PCR run. An equivocal result was defined as amplification observed crossing the fluorescence baseline threshold after or at cycle 40, a control out of range, or questions regarding sample integrity. A control that was positive for the target being tested and showed that the assay detected the presence of target DNA and that there was not PCR inhibition was a valid positive control. A control that was negative for the target being tested and showed that the reagents or the sample were not contaminated with the target prior to the testing of the sample was a valid negative control. An internal control was used to show that the extraction process was working for the purification of nucleic acid from the clinical specimen and that a negative result was truly negative and not due to an issue associated with the extraction. The following are exemplary tables:

| **Reportable Result** | **Crossing Point** | **Positive Control** | **Negative Control** | **Internal Control** | **No Template Control** | **Initial Amp No Template Control** |
|---|---|---|---|---|---|---|
| Positive Result | <40 | (+) | (-) | (+) | (-) | (-) |
| Positive Result | <40 | (-) | (-) | (+) | (-) | (-) |
| Positive Result | <40 | (+) | (-) | (-) | (-) | (-) |
| Positive Result | <40 | (-) | (-) | (-) | (-) | (-) |
| Negative Result | (-) | (+) | (-) | (+) | (-) | (-) |
| Negative Result | (-) | (+) | (+) | (+) | (-) | (-) |
| Negative Result | (-) | (-) | (+) | (+) | (-) | (-) |

| **Un-reportable Result** | **Crossing Point** | **Positive Control** | **Negative Control** | **Internal Control** | **No Template Control** | **Initial Amp No Template Control** |
|---|---|---|---|---|---|---|
| Positive Result | <40 | (+) | (+) | (+) | (+) | (+) |
| Positive Result | <40 | (-) | (+) | (+) | (+) | (+) |
| Positive Result | <40 | (+) | (+) | (-) | (+) | (+) |
| Positive Result | <40 | (-) | (+) | (-) | (+) | (+) |
| Negative Result | (-) | (-) | (-) | (+) | (+) | (+) |
| Negative Result | (-) | (+) | (-) | (-) | (+) | (+) |
| Negative Result | (-) | (+) | (+) | (-) | (+) | (+) |

### EXAMPLE 6

### VALIDATION STUDY OF INITIAL AMPLIFICATION OF A. FLAVUS

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **60.0 °C** | **0:30** | | |
| **Fast 7500 Mode** | | | | | |
| **Data Collection: Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.54207** | **34.28181** | **0.991981** | **92%** | |
| | | | | | |

| **Well** | **Sample Name** | **Detector** | **Quantity** | **Ct** | **StdDev Ct** |
|---|---|---|---|---|---|
| **A1** | 7-P2 | SYBR-Green I | 7.00E+04 | 17.4998 | 0.2227.0e+004 |
| **A2** | 7-P2 | SYBR-Green I | 7.00E+04 | 17.5704 | 0.2227.0e+004 |
| **A3** | 7-P2 | SYBR-Green I | 7.00E+04 | 17.9147 | 0.2227.0e+004 |
| **A4** | 7-P3 | SYBR-Green I | 7.00E+03 | 20.2737 | 0.08147.0e+003 |
| **A5** | 7-P3 | SYBR-Green I | 7.00E+03 | 20.1253 | 0.08147.0e+003 |
| **A6** | 7-P3 | SYBR-Green I | 7.00E+03 | 20.2576 | 0.08147.0e+003 |
| **A7** | 7-P4 | SYBR-Green I | 700 | 23.9587 | 0.1867 |
| **A8** | 7-P4 | SYBR-Green I | 700 | 23.666 | 0.1867 |
| **A9** | 7-P4 | SYBR-Green I | 700 | 24.0105 | 0.1867 |
| **A10** | 7-P5 | SYBR-Green I | 70 | 27.7824 | 0.2287 |
| **A11** | 7-P5 | SYBR-Green I | 70 | 27.3266 | 0.2287 |
| **A12** | 7-P5 | SYBR-Green I | 70 | 27.5771 | 0.2287 |
| **B1** | 7-P6 | SYBR-Green I | 7 | 31.2128 | 0.4237 |
| **B2** | 7-P6 | SYBR-Green I | 7 | 31.9752 | 0.4237 |
| **B3** | 7-P6 | SYBR-Green I | 7 | 31.9131 | 0.4237 |

The data in the above table shows shows an exemplary efficiency of the initial amplification step for *A. flavus* was 92%, and that the initial amplification step could amplify and detect as few as seven copies of fungal DNA in a single sample within the cycle limit.

### EXAMPLE 7

### VALIDATION STUDY OF INITIAL AMPLIFICATION OF A. FUMIGATUS

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.45726** | **30.68205** | **0.99059** | **95%** | |
| | | | | | |

| **Well** | **Sample Name** | **Detector** | **Quantity** | **Ct** | **StdDev Ct** |
|---|---|---|---|---|---|
| **E4** | 8-P2 | SYBR-Green I | 7.00E+04 | 14.5484 | 0.03797.0e+004 |
| **E5** | 8-P2 | SYBR-Green I | 7.00E+04 | 14.5335 | 0.03797.0e+004 |
| **E6** | 8-P2 | SYBR-Green I | 7.00E+04 | 14.6053 | 0.03797.0e+004 |
| **E7** | 8-P3 | SYBR-Green I | 7.00E+03 | 16.8545 | 0.02137.0e+003 |
| **E8** | 8-P3 | SYBR-Green I | 7.00E+03 | 16.8337 | 0.02137.0e+003 |
| **E9** | 8-P3 | SYBR-Green I | 7.00E+03 | 16.8118 | 0.02137.0e+003 |
| **E10** | 8-P4 | SYBR-Green I | 700 | 20.6416 | 0.1027 |
| **E11** | 8-P4 | SYBR-Green I | 700 | 20.5863 | 0.1027 |
| **E12** | 8-P4 | SYBR-Green I | 700 | 20.4445 | 0.1027 |
| **F1** | 8-P5 | SYBR-Green I | 70 | 23.9674 | 0.04867 |
| **F2** | 8-P5 | SYBR-Green I | 70 | 24.0335 | 0.04867 |
| **F3** | 8-P5 | SYBR-Green I | 70 | 24.0622 | 0.04867 |
| **F4** | 8-P6 | SYBR-Green I | 7 | 28.2974 | 0.1337 |
| **F5** | 8-P6 | SYBR-Green I | 7 | 28.1055 | 0.1337 |
| **F6** | 8-P6 | SYBR-Green I | 7 | 28.3616 | 0.1337 |

The data in the above table shows shows an exemplary efficiency of the initial amplification step for *A. fumigatus* was 95%, and that the initial amplification step could amplify and detect as few as seven copies of fungal DNA in a single sample within the cycle limit.

### EXAMPLE 8

### VALIDATION STUDY OF INITIAL AMPLIFICATION OF A. NIGER

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.59003** | **29.64806** | **0.996559** | **90%** | |
| | | | | | |

| **Well** | **Sample Name** | **Detector** | **Quantity** | **Ct** | **StdDev Ct** |
|---|---|---|---|---|---|
| **A7** | 3-P3 | SYBR-Green I | 7.00E+03 | 16.0678 | 0.02977.0e+003 |
| **A8** | 3-P3 | SYBR-Green I | 7.00E+03 | 16.0656 | 0.02977.0e+003 |
| **A9** | 3-P3 | SYBR-Green I | 7.00E+03 | 16.0153 | 0.02977.0e+003 |
| **A10** | 3-P4 | SYBR-Green I | 700 | 19.3052 | 0.07127 |
| **A11** | 3-P4 | SYBR-Green I | 700 | 19.1661 | 0.07127 |
| **A12** | 3-P4 | SYBR-Green I | 700 | 19.2622 | 0.07127 |
| **B1** | 3-P5 | SYBR-Green I | 70 | 22.9786 | 0.1667 |
| **B2** | 3-P5 | SYBR-Green I | 70 | 22.6919 | 0.1667 |
| **B3** | 3-P5 | SYBR-Green I | 70 | 22.6898 | 0.1667 |
| **B4** | 3-P6 | SYBR-Green I | 7 | 26.8307 | 0.1587 |
| **B5** | 3-P6 | SYBR-Green I | 7 | 26.68 | 0.1587 |
| **B6** | 3-P6 | SYBR-Green I | 7 | 26.9959 | 0.1587 |

The data in the above table shows shows an exemplary efficiency of the initial amplification step for *A. niger* was 90%, and that the initial amplification step could amplify and detect as few as seven copies of fungal DNA in a single sample within the cycle limit.

### EXAMPLE 9

### VALIDATION STUDY OF INITIAL AMPLIFICATION OF A. TERREUS

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.55094** | **32.33503** | **0.98813** | **91%** | |
| | | | | | |

| **Well** | **Sample Name** | **Detector** | **Quantity** | **Ct** | **StdDev Ct** |
|---|---|---|---|---|---|
| **G4** | 25-P2 | SYBR-Green I | 7.00E+04 | 15.2392 | 0.1197.0e+004 |
| **G5** | 25-P2 | SYBR-Green I | 7.00E+04 | 15.3296 | 0.1197.0e+004 |
| **G6** | 25-P2 | SYBR-Green I | 7.00E+04 | 15.4752 | 0.1197.0e+004 |
| **G7** | 25-P3 | SYBR-Green I | 7.00E+03 | 18.3309 | 0.09537.0e+003 |
| **G8** | 25-P3 | SYBR-Green I | 7.00E+03 | 18.2643 | 0.09537.0e+003 |
| **G9** | 25-P3 | SYBR-Green I | 7.00E+03 | 18.143 | 0.09537.0e+003 |
| **G10** | 25-P4 | SYBR-Green I | 700 | 22.4862 | 0.07037 |
| **G11** | 25-P4 | SYBR-Green I | 700 | 22.3689 | 0.07037 |
| **G12** | 25-P4 | SYBR-Green I | 700 | 22.4945 | 0.07037 |

The data in the above table shows shows an exemplary efficiency of the initial amplification step for *A. terreus* was 91%, and that the initial amplification step could amplify and detect as few as seven hundred copies of fungal DNA in a single sample within the cycle limit.

### EXAMPLE 10

### VALIDATION STUDY OF INITIAL AMPLIFICATION OF C.ALBICANS

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.23669** | **27.60403** | **0.998549** | **103%** | |
| | | | | | |

| **Well** | **Sample Name** | **Detector** | **Quantity** | **Ct** | **StdDev Ct** |
|---|---|---|---|---|---|
| **A7** | CA-P3 | SYBR-Green I | 7.00E+03 | 15.275 | 0.02747.0e+003 |
| **A8** | CA-P3 | SYBR-Green I | 7.00E+03 | 15.261 | 0.02747.0e+003 |
| **A9** | CA-P3 | SYBR-Green I | 7.00E+03 | 15.3139 | 0.02747.0e+003 |
| **A10** | CA-P4 | SYBR-Green I | 700 | 18.1125 | 0.1237 |
| **A11** | CA-P4 | SYBR-Green I | 700 | 18.2269 | 0.1237 |
| **A12** | CA-P4 | SYBR-Green I | 700 | 18.3584 | 0.1237 |
| **B1** | CA-P5 | SYBR-Green I | 70 | 21.6783 | 0.09587 |
| **B2** | CA-P5 | SYBR-Green I | 70 | 21.5857 | 0.09587 |
| **B3** | CA-P5 | SYBR-Green I | 70 | 21.4868 | 0.09587 |
| **B4** | CA-P6 | SYBR-Green I | 7 | 24.8751 | 0.1077 |
| **B5** | CA-P6 | SYBR-Green I | 7 | 24.9144 | 0.1077 |
| **B6** | CA-P6 | SYBR-Green I | 7 | 25.0762 | 0.1077 |

The data in the above table shows shows an exemplary efficiency of the initial amplification step for *C. albicans* was 103%, and that the initial amplification step could amplify and detect as few as seven copies of fungal DNA in a single sample within the cycle limit.

### EXAMPLE 11

### VALIDATION STUDY OF INITIAL AMPLIFICATION OF C.GLABRATA

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **55.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.6** | **30.756989** | **0.97** | **90%** | |
| | | | | | |

| **Well** | **Sample Name** | **Detector** | **Quantity** | **Ct** | **StdDev Ct** |
|---|---|---|---|---|---|
| **A4** | CG-P2 | SYBR-Green I | 7.00E+04 | 13.8925 | 0.007767.0e+004 |
| **A5** | CG-P2 | SYBR-Green I | 7.00E+04 | 13.8919 | 0.007767.0e+004 |
| **A6** | CG-P2 | SYBR-Green I | 7.00E+04 | 13.8787 | 0.007767.0e+004 |
| **A7** | CG-P3 | SYBR-Green I | 7.00E+03 | 15.7575 | 0.07397.0e+003 |
| **A8** | CG-P3 | SYBR-Green I | 7.00E+03 | 15.6517 | 0.07397.0e+003 |
| **A9** | CG-P3 | SYBR-Green I | 7.00E+03 | 15.794 | 0.07397.0e+003 |
| **A10** | CG-P4 | SYBR-Green I | 700 | 20.1931 | 0.09267 |
| **A11** | CG-P4 | SYBR-Green I | 700 | 20.0151 | 0.09267 |
| **A12** | CG-P4 | SYBR-Green I | 700 | 20.0594 | 0.09267 |
| **B1** | CG-P5 | SYBR-Green I | 70 | 24.8357 | 0.2957 |
| **B2** | CG-P5 | SYBR-Green I | 70 | 24.2556 | 0.2957 |
| **B3** | CG-P5 | SYBR-Green I | 70 | 24.6425 | 0.2957 |

The data in the above table shows shows an exemplary efficiency of the initial amplification step for *C. glabrata* was 90%, and that the initial amplification step could amplify and detect as few as seventy copies of fungal DNA in a single sample within the cycle limit.

### EXAMPLE 12

### VALIDATION STUDY OF INITIAL AMPLIFICATION OF C.KRUSEII

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **54.0 °C** | **0:30** | | |
| **Fast 7500 Mode** | | | | **.5uM MgCl2** | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.33876** | **27.70546** | **0.998656** | **99%** | |
| | | | | | |

| **Well** | **Sample Name** | **Detector** | **Quantity** | **Ct** | **StdDev Ct** |
|---|---|---|---|---|---|
| **E4** | CK-P3 | SYBR-Green I | 7.00E+03 | 14.9414 | 0.009417.0e+003 |
| **E5** | CK-P3 | SYBR-Green I | 7.00E+03 | 14.9602 | 0.009417.0e+003 |
| **E6** | CK-P3 | SYBR-Green I | 7.00E+03 | 14.9519 | 0.009417.0e+003 |
| **E7** | CK-P4 | SYBR-Green I | 700 | 18.0359 | 0.03117 |
| **E8** | CK-P4 | SYBR-Green I | 700 | 18.0665 | 0.03117 |
| **E9** | CK-P4 | SYBR-Green I | 700 | 18.0981 | 0.03117 |
| **E10** | CK-P5 | SYBR-Green I | 70 | 21.6067 | 0.07387 |
| **E11** | CK-P5 | SYBR-Green I | 70 | 21.6247 | 0.07387 |
| **E12** | CK-P5 | SYBR-Green I | 70 | 21.4888 | 0.07387 |
| **F1** | CK-P6 | SYBR-Green I | 7 | 24.6199 | 0.2537 |
| **F2** | CK-P6 | SYBR-Green I | 7 | 25.071 | 0.2537 |
| **F3** | CK-P6 | SYBR-Green I | 7 | 25.0437 | 0.2537 |

The data in the above table shows shows an exemplary efficiency of the initial amplification step for *C. kruseii* was 99%, and that the initial amplification step could amplify and detect as few as seven copies of fungal DNA in a single sample within the cycle limit.

### EXAMPLE 13

### VALIDATION STUDY OF INITIAL AMPLIFICATION OF C.PARAPSILOSIS

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.32038** | **28.53387** | **0.9963** | **100%** | |
| | | | | | |

| **Well** | **Sample Name** | **Detector** | **Quantity** | **Ct** | **StdDev Ct** |
|---|---|---|---|---|---|
| **A7** | CP-P3 | SYBR-Green I | 7.00E+03 | 15.9076 | 0.02887.0e+003 |
| **A8** | CP-P3 | SYBR-Green I | 7.00E+03 | 15.9472 | 0.02887.0e+003 |
| **A9** | CP-P3 | SYBR-Green I | 7.00E+03 | 15.9638 | 0.02887.0e+003 |
| **A10** | CP-P4 | SYBR-Green I | 700 | 18.9147 | 0.05447 |
| **A11** | CP-P4 | SYBR-Green I | 700 | 18.8781 | 0.05447 |
| **A12** | CP-P4 | SYBR-Green I | 700 | 18.9852 | 0.05447 |
| **B1** | CP-P5 | SYBR-Green I | 70 | 22.3079 | 0.08527 |
| **B2** | CP-P5 | SYBR-Green I | 70 | 22.1478 | 0.08527 |
| **B3** | CP-P5 | SYBR-Green I | 70 | 22.1772 | 0.08527 |
| **B4** | CP-P6 | SYBR-Green I | 7 | 25.6374 | 0.3217 |
| **B5** | CP-P6 | SYBR-Green I | 7 | 25.8345 | 0.3217 |
| **B6** | CP-P6 | SYBR-Green I | 7 | 26.2655 | 0.3217 |

The data in the above table shows shows an exemplary efficiency of the initial amplification step for *C. parapsilosis* was 100%, and that the initial amplification step could amplify and detect as few as seven copies of fungal DNA in a single sample within the cycle limit.

### EXAMPLE 14

### VALIDATION STUDY OF INITIAL AMPLIFICATION OF C.TROPICALIS

Validation studies were performed for each target. Serial dilutions containing five dilutions each were tested using the clinical assays and the APPLIED BIOSYSTEMS 7500 Fast instrument. The method described in Example 3 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **10:00** | | |
| **2** | **40** | **95.0 °C** | **0:10** | | |
| | | **60.0 °C** | **0:30** | | |
| **Fast 7500 Mode** | | | | **.5uM MgCl2** | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **SYBR** | **-3.44762** | **29.0184** | **0.998852** | **95%** | |
| | | | | | |

| **Well** | **Sample Name** | **Detector** | **Quantity** | **Ct** | **StdDev Ct** |
|---|---|---|---|---|---|
| **A4** | CT-P3 | SYBR-Green I | 7.00E+03 | 15.7819 | 0.05127.0e+003 |
| **A5** | CT-P3 | SYBR-Green I | 7.00E+03 | 15.8053 | 0.05127.0e+003 |
| **A6** | CT-P3 | SYBR-Green I | 7.00E+03 | 15.8799 | 0.05127.0e+003 |
| **A7** | CT-P4 | SYBR-Green I | 700 | 19.1597 | 0.04467 |
| **A8** | CT-P4 | SYBR-Green I | 700 | 19.0844 | 0.04467 |
| **A9** | CT-P4 | SYBR-Green I | 700 | 19.0808 | 0.04467 |
| **A10** | CT-P5 | SYBR-Green I | 70 | 22.7039 | 0.02227 |
| **A11** | CT-P5 | SYBR-Green I | 70 | 22.6636 | 0.02227 |
| **A12** | CT-P5 | SYBR-Green I | 70 | 22.6678 | 0.02227 |
| **B1** | CT-P6 | SYBR-Green I | 7 | 26.4362 | 0.2747 |
| **B2** | CT-P6 | SYBR-Green I | 7 | 25.9199 | 0.2747 |
| **B3** | CT-P6 | SYBR-Green I | 7 | 26.0171 | 0.2747 |

The data in the above table shows shows an exemplary efficiency of the initial amplification step for *C. tropicalis* was 95%, and that the initial amplification step could amplify and detect as few as seven copies of fungal DNA in a single sample within the cycle limit.

### EXAMPLE 15

### VALIDATION STUDY OF SECOND AMPLIFICATION OF A.NIGER

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.461868** | **32.216766** | **0.998178** | **94%** | |
| | | | | | |

| **Well** | **Sample Name** | **Detector** | **Quantity** | **Ct** | **StdDev Ct** |
|---|---|---|---|---|---|
| **C1** | 3 point 1 | FAM-BHQ1 | 5.45E+04 | 16.0244 | 0.1115.45e+004 |
| **C2** | 3 point 1 | FAM-BHQ1 | 5.45E+04 | 15.9009 | 0.1115.45e+004 |
| **C3** | 3 point 1 | FAM-BHQ1 | 5.45E+04 | 15.8021 | 0.1115.45e+004 |
| **C4** | 3 point 2 | FAM-BHQ1 | 5.45E+03 | 18.9988 | 0.2245.45e+003 |
| **C5** | 3 point 2 | FAM-BHQ1 | 5.45E+03 | 19.0804 | 0.2245.45e+003 |
| **C6** | 3 point 2 | FAM-BHQ1 | 5.45E+03 | 19.4208 | 0.2245.45e+003 |
| **C7** | 3 point 3 | FAM-BHQ1 | 5.45E+02 | 22.9648 | 0.226545 |
| **C8** | 3 point 3 | FAM-BHQ1 | 5.45E+02 | 22.6134 | 0.226545 |
| **C9** | 3 point 3 | FAM-BHQ1 | 5.45E+02 | 22.5418 | 0.226545 |
| **C10** | 3 point 4 | FAM-BHQ1 | 5.45E+01 | 26.1822 | 0.10254.5 |
| **C11** | 3 point 4 | FAM-BHQ1 | 5.45E+01 | 26.2426 | 0.10254.5 |
| **C12** | 3 point 4 | FAM-BHQ1 | 5.45E+01 | 26.3814 | 0.10254.5 |

The data in the above table shows shows an exemplary efficiency of the secondary amplification step for *A. nigher* was 94%, and that the initial amplification step could amplify and detect as few as 55 copies of fungal DNA in a single sample within the cycle limit.

### EXAMPLE 16

### VALIDATION STUDY OF SECOND AMPLIFICATION OF A.FLAVUS

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.553381** | **32.517784** | **0.998133** | **91%** | |
| | | | | | |

| **Well** | **Sample Name** | **Detector** | **Quantity** | **Ct** | **StdDev Ct** |
|---|---|---|---|---|---|
| **A1** | 7 point 1 | FAM-BHQ1 | 1.00E+04 | 18.4351 | 0.0161.0e+004 |
| **A2** | 7 point 1 | FAM-BHQ1 | 1.00E+04 | 18.4349 | 0.0161.0e+004 |
| **A3** | 7 point 1 | FAM-BHQ1 | 1.00E+04 | 18.4627 | 0.0161.0e+004 |
| **A4** | 7 point 2 | FAM-BHQ1 | 1.00E+03 | 21.7686 | 0.2691.0e+003 |
| **A5** | 7 point 2 | FAM-BHQ1 | 1.00E+03 | 21.3793 | 0.2691.0e+003 |
| **A6** | 7 point 2 | FAM-BHQ1 | 1.00E+03 | 21.8955 | 0.2691.0e+003 |
| **A7** | 7 point 3 | FAM-BHQ1 | 1.00E+02 | 25.3499 | 0.04571 |
| **A8** | 7 point 3 | FAM-BHQ1 | 1.00E+02 | 25.2957 | 0.04571 |
| **A9** | 7 point 3 | FAM-BHQ1 | 1.00E+02 | 25.3865 | 0.04571 |
| **A10** | 7 point 4 | FAM-BHQ1 | 1.00E+01 | 29.0791 | 0.05931 |
| **A11** | 7 point 4 | FAM-BHQ1 | 1.00E+01 | 29.1208 | 0.05931 |
| **A12** | 7 point 4 | FAM-BHQ1 | 1.00E+01 | 29.0037 | 0.05931 |

The data in the above table shows shows an exemplary efficiency of the secondary amplification step for *A. fkavys* was 91%, and that the initial amplification step could amplify and detect as few as 10 copies of fungal DNA in a single sample within the cycle limit.

### EXAMPLE 17

### VALIDATION STUDY OF SECOND AMPLIFICATION OF A.FUMIGATUS

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.514505** | **35.230759** | **0.999332** | **93%** | |
| | | | | | |

| **Well** | **Sample Name** | **Detector** | **Quantity** | **Ct** | **StdDev Ct** |
|---|---|---|---|---|---|
| **A1** | 8 P1 | FAM-BHQ1 | 6.00E+04 | 18.4053 | 0.05416.0e+004 |
| **A1** | 8 P1 | FAM-BHQ1 | 6.00E+04 | 18.4584 | 0.05416.0e+004 |
| **A2** | 8 P1 | FAM-BHQ1 | 6.00E+04 | 18.3503 | 0.05416.0e+004 |
| **A2** | 8 P2 | FAM-BHQ1 | 6.00E+03 | 21.8761 | 0.056.0e+003 |
| **A3** | 8 P2 | FAM-BHQ1 | 6.00E+03 | 21.9707 | 0.056.0e+003 |
| **A3** | 8 P2 | FAM-BHQ1 | 6.00E+03 | 21.9516 | 0.056.0e+003 |
| **A4** | 8 P3 | FAM-BHQ1 | 6.00E+02 | 25.5793 | 1.946 |
| **A4** | 8 P3 | FAM-BHQ1 | 6.00E+02 | 25.7155 | 1.946 |
| **A5** | 8 P3 | FAM-BHQ1 | 6.00E+02 | 25.2851 | 1.946 |
| **A5** | 8 P4 | FAM-BHQ1 | 6.00E+01 | 29.1491 | 1.946 |
| **A6** | 8 P4 | FAM-BHQ1 | 6.00E+01 | 29.158 | 1.946 |
| **A6** | 8 P4 | FAM-BHQ1 | 6.00E+01 | 28.8548 | 1.946 |
| **A7** | 8 P5 | FAM-BHQ1 | 6 | 32.5575 | 0.1236 |
| **A7** | 8 P5 | FAM-BHQ1 | 6 | 32.3274 | 0.1236 |
| **A8** | 8 P5 | FAM-BHQ1 | 6 | 32.3649 | 0.1236 |

The data in the above table shows shows an exemplary efficiency of the secondary amplification step for *A. fumigatus* was 93%, and that the initial amplification step could amplify and detect as few as 6 copies of fungal DNA in a single sample within the cycle limit.

### EXAMPLE 18

### VALIDATION STUDY OF SECOND AMPLIFICATION OF A. TERREUS

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.322554** | **30.472799** | **0.991658** | **100%** | |
| | | | | | |

| **Well** | **Sample Name** | **Detector** | **Quantity** | **Ct** | **StdDev Ct** |
|---|---|---|---|---|---|
| **D1** | 25p1 | FAM-BHQ1 | 5.29E+04 | 15.1743 | 0.03515.29e+004 |
| **D2** | 25p1 | FAM-BHQ1 | 5.29E+04 | 15.152 | 0.03515.29e+004 |
| **D3** | 25p1 | FAM-BHQ1 | 5.29E+04 | 15.1056 | 0.03515.29e+004 |
| **D4** | 25p2 | FAM-BHQ1 | 5.29E+03 | 17.6833 | 0.02445.29e+003 |
| **D5** | 25p2 | FAM-BHQ1 | 5.29E+03 | 17.6707 | 0.02445.29e+003 |
| **D6** | 25p2 | FAM-BHQ1 | 5.29E+03 | 17.6363 | 0.02445.29e+003 |
| **D7** | 25p3 | FAM-BHQ1 | 5.29E+02 | 21.1327 | 0.069529 |
| **D8** | 25p3 | FAM-BHQ1 | 5.29E+02 | 21.2107 | 0.069529 |
| **D9** | 25p3 | FAM-BHQ1 | 5.29E+02 | 21.2703 | 0.069529 |
| **D10** | 25p4 | FAM-BHQ1 | 5.29E+01 | 25.0073 | 0.043352.9 |
| **D11** | 25p4 | FAM-BHQ1 | 5.29E+01 | 25.0209 | 0.043352.9 |
| **D12** | 25p4 | FAM-BHQ1 | 5.29E+01 | 25.0882 | 0.043352.9 |

The data in the above table shows shows an exemplary efficiency of the secondary amplification step for *A. terreus* was 100%, and that the initial amplification step could amplify and detect as few as 53 copies of fungal DNA in a single sample within the cycle limit.

### EXAMPLE 19

### VALIDATION STUDY OF SECOND AMPLIFICATION OF C.ALBICANS

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.402701** | **34.579445** | **0.993533** | **96%** | |
| | | | | | |

| **Well** | **Sample Name** | **Detector** | **Quantity** | **Ct** | **StdDev Ct** |
|---|---|---|---|---|---|
| **C1** | CA-1 MM3 | FAM-BHQ1 | 6.18E+04 | 18.4323 | 0.05176.18e+004 |
| **C2** | CA-1 MM3 | FAM-BHQ1 | 6.18E+04 | 18.3652 | 0.05176.18e+004 |
| **C3** | CA-1 MM3 | FAM-BHQ1 | 6.18E+04 | 18.467 | 0.05176.18e+004 |
| **C4** | CA-2 MM3 | FAM-BHQ1 | 6.18E+03 | 21.4658 | 0.06876.18e+003 |
| **C5** | CA-2 MM3 | FAM-BHQ1 | 6.18E+03 | 21.3769 | 0.06876.18e+003 |
| **C6** | CA-2 MM3 | FAM-BHQ1 | 6.18E+03 | 21.3307 | 0.06876.18e+003 |
| **C7** | CA-3 MM3 | FAM-BHQ1 | 6.18E+02 | 25.2292 | 0.177618 |
| **C8** | CA-3 MM3 | FAM-BHQ1 | 6.18E+02 | 25.4023 | 0.177618 |
| **C9** | CA-3 MM3 | FAM-BHQ1 | 6.18E+02 | 25.0491 | 0.177618 |

The data in the above table shows shows an exemplary efficiency of the secondary amplification step for *C*. *albicans* was 96%, and that the initial amplification step could amplify and detect as few as 618 copies of fungal DNA in a single sample within the cycle limit.

### EXAMPLE 20

### VALIDATION STUDY OF SECOND AMPLIFICATION OF C. GLABRATA

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.490633** | **33.321709** | **0.997661** | **93%** | |
| | | | | | |

| **Well** | **Sample Name** | **Detector** | **Quantity** | **Ct** | **StdDev Ct** |
|---|---|---|---|---|---|
| **A1** | CG-1 | FAM-BHQ1 | 6.16E+04 | 16.7431 | 0.04986.16e+004 |
| **A1** | CG-1 | FAM-BHQ1 | 6.16E+04 | 16.6676 | 0.04986.16e+004 |
| **A2** | CG-1 | FAM-BHQ1 | 6.16E+04 | 16.6491 | 0.04986.16e+004 |
| **A2** | CG-2 | FAM-BHQ1 | 6.16E+03 | 19.75 | 0.146.16e+003 |
| **A3** | CG-2 | FAM-BHQ1 | 6,16E+03 | 19.8938 | 0.146.16e+003 |
| **A3** | CG-2 | FAM-BHQ1 | 6.16E+03 | 20.03 | 0.146.16e+003 |
| **A4** | CG-3 | FAM-BHQ1 | 6.16E+02 | 24.0587 | 0.278616 |
| **A4** | CG-3 | FAM-BHQ1 | 6.16E+02 | 23.5538 | 0.278616 |
| **A5** | CG-3 | FAM-BHQ1 | 6.16E+02 | 23.6028 | 0.278616 |
| **A5** | CG-4 | FAM-BHQ1 | 6.16E+01 | 26.9878 | 0.07261.6 |
| **A6** | CG-4 | FAM-BHQ1 | 6.16E+01 | 27.0094 | 0.07261.6 |
| **A6** | CG-4 | FAM-BHQ1 | 6.16E+01 | 27.1218 | 0.07261.6 |

The data in the above table shows shows an exemplary efficiency of the secondary amplification step for *C*. *glabrata* was 93%, and that the initial amplification step could amplify and detect as few as 62 copies of fungal DNA in a single sample within the cycle limit.

### EXAMPLE 21

### VALIDATION STUDY OF SECOND AMPLIFICATION OF C. KRUSEII

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.25758** | **31.336355** | **0.997822** | **102%** | |
| | | | | | |

| **Well** | **Sample Name** | **Detector** | **Quantity** | **Ct** | **StdDev Ct** |
|---|---|---|---|---|---|
| **A1** | CK-1 | FAM-BHQ1 | 1.00E+04 | 18.1957 | 0.021.0e+004 |
| **A1** | CK-1 | FAM-BHQ1 | 1.00E+04 | 18.1733 | 0.021.0e+004 |
| **A2** | CK-1 | FAM-BHQ1 | 1.00E+04 | 18.1558 | 0.021.0e+004 |
| **A2** | CK-2 | FAM-BHQ1 | 1.00E+03 | 21.7184 | 0.06631.0e+003 |
| **A3** | CK-2 | FAM-BHQ1 | 1.00E+03 | 21.6498 | 0.06631.0e+003 |
| **A3** | CK-2 | FAM-BHQ1 | 1.00E+03 | 21.5858 | 0.06631.0e+003 |
| **A4** | CK-3 | FAM-BHQ1 | 1.00E+02 | 25.0623 | 0.1091 |
| **A4** | CK-3 | FAM-BHQ1 | 1.00E+02 | 25.1351 | 0.1091 |
| **A5** | CK-3 | FAM-BHQ1 | 1.00E+02 | 24.9199 | 0.1091 |
| **AS** | CK-4 | FAM-BHQ1 | 1.00E+01 | 27.9423 | 0.0521 |
| **A6** | CK-4 | FAM-BHQ1 | 1.00E+01 | 27.845 | 0.0521 |
| **A6** | CK-4 | FAM-BHQ1 | 1.00E+01 | 27.9255 | 0,0521 |

The data in the above table shows shows an exemplary efficiency of the secondary amplification step for *C*. *kruseii* was 102%, and that the initial amplification step could amplify and detect as few as ten copies of fungal DNA in a single sample within the cycle limit.

### EXAMPLE 22

### VALIDATION STUDY OF SECOND AMPLIFICATION OF C. TROPICALIS

Validation studies were performed for each target. Serial dilutions containing five dilutions each were tested using the clinical assays and the APPLIED BIOSYSTEMS 7500 Fast instrument. The method described in Example 5 was used to obtain the data in the following table:

| **Instrument Type: Applied Biosystems 7500 Fast Real-Time PCR System** | | | | | |
|---|---|---|---|---|---|
| **Thermal Cycler Profile** | | | | | |
| **Stage** | **Repetitions** | **Temperature** | **Time** | | |
| **1** | **1** | **95.0 °C** | **0:20** | | |
| **2** | **40** | **95.0 °C** | **0:03** | | |
| | | **60.0 °C** | **0:30** | | |
| **Fast 7500 Mode** | | | | | |
| **Data Collection : Stage 2 Step 2** | | | | | |
| **PCR Volume: 20 µL** | | | | | |
| | | | | | |
| **Standard Curve Information** | | | | | |
| **Detector Name** | **Slope** | **Intercept** | **R2** | **% Efficiency** | |
| **FAM-BHQ1** | **-3.484473** | **29.33049** | **0.995491** | **94%** | |
| | | | | | |

| **Well** | **Sample Name** | **Detector** | **Quantity** | **Ct** | **StdDev Ct** |
|---|---|---|---|---|---|
| **G1** | CT-1 MM4 | FAM-BHQ1 | 1.00E+04 | 15,5726 | 0.08141.0e+004 |
| **G2** | CT-1 MM4 | FAM-BHQ1 | 1.00E+04 | 15.735 | 0.08141.0e+004 |
| **G3** | CT-1 MM4 | FAM-BHQ1 | 1.00E+04 | 15.6642 | 0.08141.0e+004 |
| **G4** | CT-2 MM4 | FAM-BHQ1 | 1.00E+03 | 18.4636 | 0.1131.0e+003 |
| **G5** | CT-2 MM4 | FAM-BHQ1 | 1.00E+03 | 18.6878 | 0.1131.0e+003 |
| **G6** | CT-2 MM4 | FAM-BHQ1 | 1.00E+03 | 18.5577 | 0.1131.0e+003 |
| **G7** | CT-3 MM4 | FAM-BHQ1 | 1.00E+02 | 22.1231 | 0.05171 |
| **G8** | CT-3 MM4 | FAM-BHQ1 | 1.00E+02 | 22.2052 | 0.05171 |
| **G9** | CT-3 MM4 | FAM-BHQ1 | 1.00E+02 | 22.2186 | 0.05171 |
| **G10** | CT-4 MM4 | FAM-BHQ1 | 1.00E+01 | 25.9278 | 0.1471 |
| **G11** | CT-4 MM4 | FAM-BHQ1 | 1.00E+01 | 26.0553 | 0.1471 |
| **G12** | CT-4 MM4 | FAM-BHQ1 | 1.00E+01 | 26.2208 | 0.1471 |

The data in the above table shows shows an exemplary efficiency of the secondary amplification step for *C*. *tropicalis* was 94%, and that the initial amplification step could amplify and detect as few as ten copies of fungal DNA in a single sample within the cycle limit.

### EXAMPLE 23

### PROCESS VALIDATION PROTOCOL

The APPLIED BIOSYSTEMS® 7500 Fast system was used with specially designed target probes and primers to detect DNA in clinical samples utilizing real-time PCR technology. Qualitative detection of fungal DNA was generated from fourteen organisms in clinical samples.

The following represents the exemplary flow of a clinical sample from extraction to reporting.

The clinical sample was extracted utilizing a commercial extraction kit. Following the extraction, PCR reactions were setup in triplicate with the extracted nucleic acid from the clinical sample (and positive and negative control) and each of the target probe and primer set. The 96 well plate containing the sample reactions was placed on the APPLIED BIOSYSTEMS® 7500 Fast instrument and a preprogrammed real-time program was run and data collected by the instrument. After the instrument run was complete, the data was analyzed to determine if the run was acceptable and to determine if the sample was positive or negative for the target tested. Upon completion of this analysis, a report was generated for the sample processed and the results of the testing was reviewed, approved, and reported. A run was determined to be acceptable if the internal control (GEO) for the extracted sample was positive, the Positive Control was positive, the Negative Control was negative, the No Template control was negative, and no instrument errors were generated during the course of a PCR run.

### Validation Protocol:

Materials and Methods: QIAGEN QIAAMP® Mini Kit Extraction Procedure; APPLIED BIOSYSTEMS® 7500 Fast Real-Time PCR Procedure; Fugal Spore Detection Utilizing Real-Time PCR (7500Fast).

### Test Samples:

∘ Fugal Target DNA Control Samples
   ▪ Control Samples for targets:
      - 1 S chartarum (Stachybotrys)
      - 3 A niger (Aspergillus)
      - 4 P chrysogenium (Penicillium)
      - 5 P verrucosum (Penicillium)
      - 6 GEO (Control)
      - 7 A Flavus (Aspergillus)
      - 8 A fumigatus (Aspergillus)
      - 14 F solani (Fusarium)
      - 23 S echinata (Stachybotrys)
      - 25 A terreus (Aspergillus)
      - CA C albicans (Candida)
      - CT C tropicalis (Candida)
      - CKC kruseii (Candida)
      - CGC glabrata (Candida)
∘ Blinded Spiked Validation Samples
   ▪ 10 Fungal Spiked Samples
      - Sample 1 (Spiked with Targets 3, 8)
      - Sample 2 (Spiked with Targets 3,4)
      - Sample 3 (Spiked with Targets 1,6,14,25)
      - Sample 4 (Spiked with Targets 1, 4, 5, 23)
      - Sample 5 (Spiked with Targets 4, 7, 23)
      - Sample 6 (Spiked with No Targets)
      - Sample 7 (Spiked with Targets 1, 23)
      - Sample 8 (Spiked with Targets 4, 8, 25)
      - Sample 9 (Spiked with Targets 3, 7)
      - Sample 10 (Spiked with Targets 4, 23)
   ▪ 10 Candida Spiked Samples
      - Sample 11 (Spiked with Targets CA, CG)
      - Sample 12 (Spiked with Targets CT, CK)
      - Sample 13 (Spiked with Targets CA, CG)
      - Sample 14 (Spiked with No Targets)
      - Sample 15 (Spiked with Targets CT, CG, CK)
      - Sample 16 (Spiked with Targets CA, CT, CG, CK)
      - Sample 17 (Spiked with Targets CT)
      - Sample 18 (Spiked with Targets CK)
      - Sample 19 (Spiked with Targets CA)
      - Sample 20 (Spiked with Targets CG, CK)
∘ Samples run on the Cepheid SmartCycler
   ▪ Aspergillus Samples (Accession Number)
      - 150939 (BAL)
      - 154018 (Sputum)
      - 147499 (Sputum)
      - 122074 (Tissue)
      - 134404 (BAL)
      - 131746 (BAL)
      - 127675 (Tissue)
      - 126482 (Tissue)
   ▪ Penicillium Samples
      - 150930 (Sputum)
      - 154017 (Nasal Wash)
      - 147498 (Nasal Wash))
      - 139270 (BAL))
      - 134403 (Tissue)
      - 131747 (BAL)
      - 122073 (BAL)
      - 126483 (BAL)
   ▪ Stachy/Fusarium Samples
      - 150935 (Nasal Wash)
      - 154016 (BAL)
      - 147496 (Sputum)
      - 139269 (Tissue)
      - 134402 (Nasal Wash)
      - 131748 (BAL)
      - 131749 (BAL)
      - 127677 (Tissue)
      - 126484 (Nasal Wash)
   ▪ Candida Samples
      - 150934 (Urine)
      - 154014 (Urine)
      - 139883 (Urine)
      - 139268 (Urine)
      - 134405 (Urine)
      - 131750 (Urine)
      - 127678 (Urine)
      - 126485 (Urine)

Testing Conditions: Real-time PCR was performed utilizing fungal target DNA for all targets to determine cycle cutoff (Established Cycle Threshold (Ct) Value - Qualitative Analysis). Real-time PCR was performed utilizing the fungal target DNA for all targets on a 4 point dilution curve in triplicate (Assay Optimization). The runs accessed the following: Linear Standard Curve, High Amplification Efficiency, and Consistency across replicate reactions.

Blinded spiked samples were tested and the presence of interfering substances was determined (e.g., interference from different tubes and plastic ware, reagents, instrument conditions, etc.). All fungal assays were performed on blinded spiked samples 1 through 10 to determine if the DNA spiked in the sample could be detected by the assays. All candida assays were run on blinded spiked samples 11 through 20 to determine if the DNA spiked in the samples could be detected by the assays. Ten fungal assays were run with 10 blinded samples. Four candida assays were run with 10 blinded samples. All samples tested on the CEPHEID SMARTCYCLER® utilizing specific fungal and candida assays were run utilizing the same assay and sample on the APPLIED BIOSYSTEMS® 7500 instrument.

Data Collected: The Cycle Threshold (Ct) value for each sample tested was collected and recorded. The last cycle was approved for detection of a positive qualitative result. Standard curves were generated for all fungal and candida assays. Data was collected, analyzed, and recorded for the following performance characteristics: Linear Standard Curve, High Amplification Efficiency, and Consistency across replicate reactions. For assays run with spiked blinded samples, data showed accuracy and specificity of the assays: 10 fungal assays run with 10 blinded samples; 4 candida assays run with 10 blinded samples. For assays run with previously tested samples, data matched previous runs on the CEPHEID SMARTCYCLER®. The data confirmed test sample types, BAL, Nasal Wash, Sputum, tissue, and urine (Candida only). The data showed no interfering substances with the use of various plastic ware, reagents, or instrument conditions.

Test samples run on the CEPHEID SMARTCYCLER® were tested on the APPLIED BIOSYSTEMS® 7500 instrument:
▪ Aspergillus Samples (Accession Number)
   - 150939 (BAL)
   - 154018 (Sputum)
   - 147499 (Sputum)
   - 122074 (Tissue)
   - 134404 (BAL)
   - 131746 (BAL)
   - 127675 (Tissue)
   - 126482 (Tissue)
▪ Penicillium Samples
   - 150930 (Sputum)
   - 154017 (Nasal Wash)
   - 147498 (Nasal Wash))
   - 139270 (BAL))
   - 134403 (Tissue)
   - 131747 (BAL)
   - 122073 (BAL)
   - 126483 (BAL)
▪ Stachy/Fusarium Samples
   - 150935 (Nasal Wash)
   - 154016 (BAL)
   - 147496 (Sputum)
   - 139269 (Tissue)
   - 134402 (Nasal Wash)
   - 131748 (BAL)
   - 131749 (BAL)
   - 127677 (Tissue)
   - 126484 (Nasal Wash)
▪ Candida Samples
   - 150934 (Urine)
   - 154014 (Urine)
   - 139883 (Urine)
   - 139268 (Urine)
   - 134405 (Urine)
   - 131750 (Urine)
   - 127678 (Urine)
   - 126485 (Urine)

Acceptance Criteria: A positive result was defined as any amplification observed crossing a baseline fluorescence between cycles 1 and 39 of the real-time PCR run. A negative result was defined as no amplification observed crossing a baseline fluorescence between cycles 1 and 39 of the PCR run. (See note regarding diluted spore stocks). A positive control included a control that was positive for the target being tested and showed that the assay would show a positive in the presence of target spores and that there was not PCR inhibition. (Note: a sample that showed amplification for a target when the positive control was negative could be reported as a positive result.). A negative control included a control that was negative for the target being tested and showed that the reagents or the sample were not contaminated with the target prior to the testing of the sample. (Note: a sample that showed amplification at an earlier cycle than a contaminated negative control could be reported as a due to the fact that the contamination cannot cause a sample to report a stronger positive than the contamination.). An internal control included a control used to show that the extraction process was working fine for the purification of nucleic acid from the clinical specimen and that a negative result was truly negative and not due to an issue associated with the extraction. (Note: the internal control must be positive for any sample to be reported as negative for a target.)

Assay Optimization and Conclusions: Qualitative Analysis Real-time PCR was performed utilizing fungal target DNA for all targets to determine cycle cutoff (Establish Cycle Threshold (Ct) Value). The qualitative cutoff for this assay for a positive detection was a crossover threshold of 39.

All fungal and candida targets had a Linear Standard Curve - R² > 0.980. All fungal and candida targets had a High Amplification Efficiency - 90-105%. All fungal and candida targets had Consistency across replicate reactions and there weas no clinically significant variation between the replicates.

All assays detected the appropriate spiked samples in all 20 spiked samples tested. The testing of each spiked fungal blinded sample with fungal assays on the APPLIED BIOSYSTEMS® 7500 instrument detected the appropriate target for that sample. The testing of each spiked candida blinded sample with candida assays on the APPLIED BIOSYSTEMS® 7500 instrument detected the appropriate target for that sample. No interference was detected.

All sample results from samples tested on the APPLIED BIOSYSTEMS® 7500 instrument matched the results generated from the CEPHEID SMARTCYCLER® with the exception of the following:
1. 134404 (BAL) - A. Flavus (Target 7) was not detected in Sample 134404 by the CEPHEID SMARTCYCLER® but was detected by the APPLIED BIOSYSTEMS® 7500 instrument. Upon review of the data it was determined that improved sensitivity by the APPLIED BIOSYSTEMS® 7500 system enabled the system to detect the target in the BAL sample.
2. 154017 (Nasal Wash) - P. Chrysogenium (Target 4) was not detected in Sample 154017 by the CEPHEID SMARTCYCLER® but was detected by the APPLIED BIOSYSTEMS® 7500 instrument. Upon review of the data it was determined that improved sensitivity by the APPLIED BIOSYSTEMS® 7500 system enabled the system to detect the target in the Nasal Wash sample.
3. 126485 (Urine) - C. Krusei (Target CK) was not detected in Sample 126485 by the CEPHEID SMARTCYCLER® but was detected by the APPLIED BIOSYSTEMS® 7500 instrument. Upon review of the data it was determined that improved sensitivity by the APPLIED BIOSYSTEMS® 7500 system enabled the system to detect the target in the Urine sample.
4. 127675 (Tissue) - A terreus (Target 25) was not detected by the APPLIED BIOSYSTEMS® 7500 instrument but was detected by the CEPHEID SMARTCYCLER®. It was determined to be a problem with sample degradation.
5. 139269 (Tissue) - S echinata (Target 23) was not detected by the APPLIED BIOSYSTEMS® 7500 instrument but was detected by the CEPHEID SMARTCYCLER®. It was determined to be a problem with sample degradation.
6. 139268 (Urine) - C glabrata (Target CG) was not detected by the APPLIED BIOSYSTEMS® 7500 instrument but was detected by the CEPHEID SMARTCYCLER®. It was determined to be a problem with sample degradation.

Results produced in this validation for all fungal and candida assays were found acceptable to validate these assays for testing on the APPLIED BIOSYSTEMS® 7500 instrument.

### EXAMPLE 24

### COMPARING PCR INSTRUMENTS

The specially designed secondary primers and probes were tested with two different PCR instruments to validate and demonstrate the qualitative detection of the primers and probes. The analysis was done using the method described in Example 23. The two different instruments shown below, SMART CYCLER II ® and 7500 Fast use different reagents, hardware, and software to amplify a strand of DNA.

| **Aspergillus Panel** | | | | | | | | | |
|---|---|---|---|---|---|---|---|---|---|
| **Accession** | **Sample** | **3 Smart** | **3 7500F** | **7 Smart** | **7 7500F** | **8 Smart** | **8 7500F** | **25 Smart** | **25 7500F** |
| **150939** | **BAL** | ND | ND | ND | ND | ND | ND | Detected | Detected |
| **154018** | **Sputum** | ND | ND | ND | ND | ND | ND | Detected | Detected |
| **147499** | **Sputum** | ND | ND | ND | ND | Detected | Detected | Detected | Detected |
| **122074** | **Tissue** | ND | ND | Detected | Detected | Detected | Detected | Detected | Detected |
| **134404** | **BAL** | Detected | Detected | ND | Detected | ND | ND | Detected | Detected |
| **131746** | **BAL** | ND | ND | ND | ND | ND | ND | Detected | Detected |
| **127675** | **Tissue** | ND | ND | Detected | Detected | ND | ND | Detected | ND |
| **126482** | **Tissue** | ND | ND | Detected | Detected | ND | ND | Detected | Detected |

| **Penicillium Panel** | | | | | |
|---|---|---|---|---|---|
| **Accession** | **Sample** | **4 Smart** | **47500F** | **5 Smart** | **5 7500F** |
| **150930** | **Sputum** | Detected | Detected | ND | ND |
| **154017** | **Nasal Wash** | ND | Detected | ND | ND |
| **147498** | **Nasal Wash** | Detected | Detected | Detected | Detected |
| **139270** | **BAL** | Detected | Detected | Detected | Detected |
| **134403** | **Tissue** | Detected | Detected | Detected | Detected |
| **131747** | **BAL** | Detected | Detected | Detected | Detected |
| **122073** | **BAL** | Detected | Detected | ND | ND |
| **126483** | **BAL** | ND | ND | ND | ND |

| **Stachybotrys Panel** | | | | | | | | | |
|---|---|---|---|---|---|---|---|---|---|
| **Accession** | **Sample** | **1 Smart** | **1 7500F** | **23 Smart** | **23 7500F** | **14 Smart** | **14 7500F** | **6 Smart** | **6 7500F** |
| **150935** | **Nasal Wash** | Detected | Detected | Detected | Detected | ND | ND | Detected | Detected |
| **154016** | **BAL** | Detected | Detected | Detected | Detected | ND | ND | Detected | Detected |
| **147496** | **Sputum** | ND | ND | ND | ND | ND | ND | Detected | Detected |
| **139269** | **Tissue** | Detected | Detected | Detected | ND | ND | ND | Detected | Detected |
| **134402** | **Nasal Wash** | ND | ND | ND | ND | ND | ND | Detected | Detected |
| **131748** | **BAL** | Detected | Detected | Detected | Detected | ND | ND | Detected | Detected |
| **131749** | **BAL** | ND | ND | ND | ND | ND | ND | Detected | Detected |
| **127677** | **Tissue** | ND | ND | ND | ND | ND | ND | Detected | Detected |
| **126484** | **Nasal Wash** | ND | ND | Detected | Detected | ND | ND | Detected | Detected |

| **Candida Panel** | | | | | | | | | |
|---|---|---|---|---|---|---|---|---|---|
| **Accession** | **Sample** | **CA Smart** | **CA 7500F** | **CT Smart** | **CT 7500F** | **CK Smart** | **CK 7500F** | **CG Smart** | **CG 7500F** |
| **15093A** | **Urine** | ND | ND | ND | ND | Detected | Detected | Detected | Detected |
| **154014** | **Urine** | Detected | Detected | ND | ND | ND | ND | Detected | Detected |
| **139883** | **Urine** | Detected | Detected | ND | ND | ND | ND | ND | ND |
| **139268** | **Urine** | ND | ND | ND | ND | Detected | Detected | Detected | ND |
| **134405** | **Urine** | Detected | Detected | Detected | Detected | Detected | Detected | Detected | Detected |
| **131750** | **Urine** | ND | ND | Detected | Detected | Detected | Detected | Detected | Detected |
| **127678** | **Urine** | Detected | Detected | Detected | Detected | Detected | Detected | Detected | Detected |
| **126485** | **Urine** | ND | ND | Detected | Detected | ND | Detected | ND | ND |

The data illustrates that the specially designed primers were able to detect the presence of fungal DNA in various patient samples using the two different PCR instruments.

### EXAMPLE 25

### SPECIFICITY

The secondary primers and probes were tested to demonstrate specificity for their targets. The data below was obtained using methods described in Example 23. The primers and probes were able to successfully amplify and detect their own target and avoid non-specific interactions. The primers and probes were able to do this with multiple fungal strains of DNA present in the sample. All fungal assays were performed on blinded spiked samples 1 through 10 to determine if the DNA spiked in the sample could be detected by the assays. All candida assays were run on blinded spiked samples 11 through 20 to determine if the DNA spiked in the samples could be detected by the assays. Ten fungal assays were run with 10 blinded samples. Four candida assays were run with 10 blinded samples. All samples tested on the CEPHEID SMARTCYCLER® utilizing specific fungal and candida assays were run utilizing the same assay and sample on the APPLIED BIOSYSTEMS® 7500 instrument.

| | **Target** | | | | | | | | | | | | | |
|---|---|---|---|---|---|---|---|---|---|---|---|---|---|---|
| **Validation Sample** | **1** | **3** | **4** | **5** | **6** | **7** | **8** | **14** | **23** | **25** | **CA** | **CT** | **CG** | **CK** |
| **1** | | X | | | | | X | | | | | | | |
| **2** | | X | X | | | | | | | | | | | |
| **3** | X | | | | X | | | X | | X | | | | |
| **4** | X | | X | X | | | | | X | | | | | |
| **5** | | | X | | | X | | | X | | | | | |
| **6** | | | | | | | | | | | | | | |
| **7** | X | | | | | | | | X | | | | | |
| **8** | | | X | | | | X | | | X | | | | |
| **9** | | X | | | | X | | | | | | | | |
| **10** | | | X | | | | | | X | | | | | |
| **11** | | | | | | | | | | | X | | X | |
| **12** | | | | | | | | | | | | X | | X |
| **13** | | | | | | | | | | | X | | X | |
| **14** | | | | | | | | | | | | | | |
| **15** | | | | | | | | | | | | X | X | X |
| **16** | | | | | | | | | | | X | X | X | X |
| **17** | | | | | | | | | | | | X | | |
| **18** | | | | | | | | | | | | | | X |
| **19** | | | | | | | | | | | X | | | |
| **20** | | | | | | | | | | | | | X | X |

| **Fungal Target** | **Fungal Organism** | **Candida Target** | **Candida Organism** |
|---|---|---|---|
| **1** | **Stachybotrys chartarum** | **CA** | **Candida albicans** |
| **3** | **Aspergillus niger** | **CT** | **Candida tropicalis** |
| **4** | **Penicillium chrysogenum** | **CG** | **Candida glabrata** |
| **5** | **Penicillium verrucosum** | **CK** | **Candida kruseii** |
| **6** | **Geo** | | |
| **7** | **Aspergillus flavus** | | |
| **8** | **Aspergillus fumigatus** | | |
| **14** | **F solani** | | |
| **23** | **Stachybotrys echinata** | | |
| **25** | **Aspergillus terreus** | | |

For assays run with spiked blinded samples, data showed accuracy and specificity of the assays: 10 fungal assays run with 10 blinded samples; 4 candida assays run with 10 blinded samples. For assays run with previously tested samples, data matched previous runs on the CEPHEID SMARTCYCLER®. The data confirmed test sample types, BAL, Nasal Wash, Sputum, tissue, and urine (Candida only). The data showed no interfering substances with the use of various plastic ware, reagents, or instrument conditions.

### EXAMPLE 26

### SMART CYCLER PROTOCOL

The CEPHEID SMARTCYCLER® system is an integrated DNA/RNA amplification and detection instrument system based on the proprietary microprocessor-controlled I-CORE® module. Ease of use is designed into the system through the Smart Cycler software. Each Smart Cycler II processing block contains sixteen independently controlled, programmable I-CORE modules, each with one reaction site. Thermally optimized proprietary reaction tubes combined with the unique design of the I-CORE modules allow for rapid cycling and faster amplification and detection. A total of six Smart Cycler II processing blocks can be daisy-chained together, allowing simultaneous custom analysis of 96 discrete samples.

The Smart cycler II is ideally suited to research, such as PCR, and RT-PCR, that requires automatic, repeated, rapid heating and cooling cycles for test samples. Specific sequences can be detected using hybridization probes or intercalating dyes. The system has the capacity to store a number of user-generated protocols. All data, including cycling programs and assay results, can be stored in a database. Selected data can be exported to spreadsheet programs.

The disposable reaction tube is inserted into the I-CORE thermal cycling module for amplification. The chamber includes two heater plates made of a ceramic material that has high thermal conductivity to assure temperature uniformity and rapid heat transfer. Resistive heater elements are deposited on the ceramic plates using thick film technologies and a thermistor attached directly to each plate monitors it temperature. Cooling is accomplished with a high-efficiency fan that moves ambient air across the heater plates. The thermal cycling chamber's temperature is controlled by the instrument's firmware. The firmware incorporates a control loop to ensure rapid heating of the plates and to control their temperature overshoot around the desired point, allowing the temperature of the fluid in the reaction tube to be changed rapidly and precisely.

The Smart Cycler II optical system uses high intensity light-emitting diodes (LEDs), silicon photodetectors, and appropriate filter for excitation and detection of four different spectral bands. The optical system includes two optical blocks: (1) a four-color excitor module and (2) a four-color detector module. These blocks are positioned within the device such that their apertures mate with the optical windows of the reaction tube, allowing excitation and emission detection of the reaction mixture (Cycler II Operator Manual; Cepheid, 904 Caribbean Drive Sunnyvale, CA 94089, 888-838-3222).

### EXAMPLE 27

### FUNGAL SPORE DETECTION PROTOCOL

The qualitative detection of the following fungal targets was performed.

| **Aspergillus** | **Penicillium** | **Stachybotrys** | **Fusarium** | **Candida** | **Control** |
|---|---|---|---|---|---|
| (3) niger | (4) chrysogenium | (1) chartarum | (14) solani | (CA) albicans | (6) GEO |
| (7) flavus | (5) verrucosum | (23) echinata | | (CK) kruseii | |
| (8) fumigatus | | | | (CG )glabrata | |
| (25) A terreus | | | | (CT )tropicalis | |

**Specimens:** Nucleic acid samples were extracted (e.g., from tissue, paraffin embedded tissue, swabs, spore solutions, and/or urine, etc.). A minimum of 25.0 mg or 200.0 µL of sample was used for the extraction. DNA specimens could be stored at -10 to -25.9°C until processed.

**Materials:** SmartMix HM PCR Beads (Store at 2-8° C, expiration per manufacturer) (cat no. SMHM1-100N-200) (Cepheid, 904 Caribbean DriveSunnyvale, CA 94089, 888-838-3222); PCR grade purified water; Smart Cycler® Reaction Tubes (cat no. 9000022) (Cepheid, 904 Caribbean Drive Sunnyvale, CA 94089, 888-838-3222); 1.5 mL tubes; Barrier pipette tips capable of 0.1µL-1000.0µL volumes; Disposable gloves; Marker; Smart Cycler® II Instrument (Cepheid, 904 Caribbean Drive Sunnyvale, CA 94089, 888-838-3222); Smart Cycler® II cooling block with plastic rack (Cepheid, 904 Caribbean Drive Sunnyvale, CA 94089, 888-838-3222);

Smart Cycler® II modified centrifuge (Cepheid, 904 Caribbean Drive Sunnyvale, CA 94089, 888-838-3222); Microcentrifuge for 1.5mL tubes; Tube puller (Cepheid, 904 Caribbean Drive Sunnyvale, CA 94089, 888-838-3222); Tube racks capable of holding 1.5 mL tubes; Filtered PCR workstation; Pipettes capable of volumes ranging from 5.0 µL to 1000.0 µL; Primers and Probes (Store at room temperature indefinitely while lyophilized, Store at -10 to -28°C upon reconstitution and dilution of probes, primers and working stocks (Integrated DNA Technologies, Inc., orders@idtdna.com, 800-328-2661). Note: Probes were synthesized with the reporter FAM attached to the 5' end of the probe and the quencher BHQ attached to the 3' end of the probe. Primers were synthesized at a scale of 25 nm and probes are synthesized at a scale of 100 nm. Sequences were as follows:
**Target 1** - *S chartarum*
   Probe 1 char: 5'-ttgcttcggcgggaacgccccg
   Primer F2: 5'-gcggagggatcattaccgag
   Primer R2: 5'-atcgatgccagagccaagag
**Target 3 -** *A niger*
   Probe 3 niger: 5'-tgtctattgtaccctgttgcttc
   Primer F1: 5'-cgtaggtgaacctgcggaag
   Primer R1: 5'-atcgatgccggaaccaagag
**Target 4** *- P chrysogenum*
   Probe 4 chry: 5'-ctctgtctgaagattgtagtctgagt
   Primer F1: 5'-cgtaggtgaacctgcggaag
   Primer R1: 5'-atcgatgccggaaccaagag
**Target 5** *- P verrucosum*
   Probe 5 verru: 5'-cccgcctttgctggccgcc
   Primer F1: 5'-cgtaggtgaacctgcggaag
   Primer R1: 5'-atcgatgccggaaccaagag
**Target 6 -** G candidum
   For Geo F1H: 5'-ggatctcttggttctcgtatc
   Rev Geo R1H: 5'-cttgatctgaggttgaatagtg
   Probe 6 geo: 5"-aacgcacattgcactttggggtatc
**Target 7 -** A *flavus*
   Probe 7 flav: 5'-cccgccattcatggccgccggg
   Primer F1: 5'-cgtaggtgaacctgcggaag
   Primer R1: 5'-atcgatgccggaaccaagag
**Target 8 -** *A fumigatus*
   Probe 8 fumi: 5'-aaagtatgcagtctgagttgattatc
   Primer F1: 5'-cgtaggtgaacctgcggaag
   Primer R1: 5'-atcgatgccggaaccaagag
**Target 14 -** F *solani*
   Probe 14 salani: 5'-cgggaatagacggccccgtgaaac
   Primer F2: 5'-gcggagggatcattaccgag
   Primer R2: 5'-atcgatgccagagccaagag
**Target 23 -** S *echinata*
   Probe 23 echin: 5'-ttgcttcggcgggagagccccg
   Primer F2: 5'-gcggagggatcattaccgag
   Primer R2: 5'-atcgatgccagagccaagag
**Target 25 -** A terreus
   Probe 25: 5"- AGTCTGAGTGTGATTCTTTGCAATC
   Primer 25 F: 5"-ACATGAACCCTGTTCTGAAAG
   Primer 25 R: 5"-CCAAGAGATCCATTGTTGAAAG
**Target CA -** C albicans
   Probe CA: 5' -TCGGGGGCGGCCGCTGCGG
   Primer CA F: 5" -AAAAAGTACGTGAAATTGTTG
   Primer CA R: 5" -AAGCCGTGCCACATTC
**Target CK -** C kruseii
   Probe CK: 5" -AAGGCGGTGTCCAAGTCCCTTG
   Primer CK F: 5" -TCAGTAGCGGCGAGTGAAG
   Primer CK R: 5" -AGAAGGGCCTCACTGCTTC
**Target CG -** C glabrata
   Probe CG: 5" -ACCTAGGGAATGTGGCTCTGCG
   Primer CG F: 5" -TGGGCCAGCATCGGTTTTG
   Primer CG R: 5" -CCTAGATAACAAGTATCGCAG
**Target CT -** C tropicalis
   Probe CT: 5" -TCGGGGGTGGCCTCTACAG
   Primer CT F: 5" -AAAAAGTACGTGAAATTGTTG
   Primer CT R: 5" - AAGCCGTGCCACATTC

**Validation and Proficiency Testing**: All fungal targets were validated for qualitative determination. Proficiency testing of these fungal targets was processed as decribed. Every clinical sample processed was inoculated with spores from the internal control target Geo to show that a negative target result is a true negative result and not related to the extraction of the sample. The samples were processed through the extraction protocol and amplified and detected utilizing primer and probes specific for Geometrica. A positive control (e.g., extracted from tissue, spore solutions, or purchased from a vender) for each target of interest (Primer/Probe sets) was processed along with each clinical sample in each real-time PCR runThe positive control showed that the primer/probe set for each target is not being inhibited and showed that a negative result is a true negative. A negative control (e.g., extracted from tissue or water) for each target of interest (Primer/Probe sets) was processed along with each clinical sample in each real-time PCR run. The negative control showed that the primer/probe set, water and extraction reagents for each target is not contaminated with the target and showed that a positive result is a true positive.

**Reagent Lots:** Primer/Probe, SmartMix, Negative and Positive Control Lots are tested in parallel with kit lots currently in use. In order to test the new lot, one sample from the test batch was processed twice in the same protocol run using both the new reagent lot and the old reagent lot. After the test was completed, the new lot of reagents was labeled with "QC Date," the date of the test, and the initials of the person conducting the test. Record extraction results for the lot test in the Reagent Lot Log (10.7 G). If the results from the new lot differed from the current lot, the lot test was repeated or the reagent was disgarded. All lot numbers and lot test results were recorded in the Reagent Lot Log (10.7 G).

### Procedure: Oligo and Probe Working Stock Preparation and Reaction Setup

Dilution of Probe Stocks: The lyophilized probes were resuspended in PCR grade water to a final concentration of 100 uM. (Example: If the synthesis yields 15.03 nMoles, add 150.3 of PCR grade water to achieve 100uM concentration)

Dilution of Primer Stocks: The lyophilized primers were resuspended in PCR grade water to a final concentration of 100 uM. (Example: If the synthesis yields 38.6 nMoles, add 386 uL of PCR grade water to achieve 100uM concentration)

**Exemplary Reaction Setup:** The reaction setup for one reaction is shown below. In some cases the addition of MgCl₂ or varying concentrations of primer/probe mix was required for PCR.

| | |
|---|---|
| **DNA** | 5.0 uL |
| **Primer/Probe Working Stock** | 3.5 uL |
| **SmartMix Beads** | 0.5 Beads |
| **PCR Grade Water** | 16.5 uL |
| **Total** | **25.0 uL** |

Smart Cycler Cycling Parameters (Omni Fungal I): Omni Fungal I was the primary program used for the fungal real time assays and the run parameters for this program are outlined below. Cases may occur where changes to this program may be necessary for a specific target or specimen type.

### Step 1 (1 Cycle)

| | |
|---|---|
| **Hot Start:** | 95°C for 120 seconds |

### Step 2 (45 Cycles)

| | |
|---|---|
| **Denature:** | 95°C for 5 seconds |
| **Anneal:** | 60°C 45 seconds |

**SmartCycler Master Mix Worksheet**

**I. Run Information**

| | | | |
|---|---|---|---|
| **Test** | Omni Fungal I | **Negative Control Lot No.** | 052005 |
| **Run No.** | 061205.1 | **Positive Control Lot No.** | 052705 |
| **Date** | 6/18/05 | **Internal Control Lot No.** | 053005 |
| **Initials** | JSS | | |

**II. Master Mix Setup**

| **Set 1** | **Reagent** | **Lot #** | **Volume (uL)** | **Reaction No.** | **Total Amount** | **Target** |
|---|---|---|---|---|---|---|
| **1** | H2O | 4532 | 16.5 | 6 | 99.0 | (3) A niger |
| **2** | P/P Working Stock | 040505 | 3.5 | 6 | 21.0 | |
| **3** | SmartMix (Bead) | 2456 | 0.5 | 6 | 3.0 | |
| **4** | MgCl2 | NA | 0.0 | 0 | 0.0 | |

| **Set 2** | **Reagent** | **Lot #** | **Volume (uL)** | **Reaction No.** | **Total Amount** | **Target** |
|---|---|---|---|---|---|---|
| **1** | H2O | 4532 | 16.5 | 6 | 99.0 | (2) A Versicolor |
| **2** | P/P Working Stock | 020705 | 3.5 | 6 | 21.0 | |
| **3** | SmartMix (Bead) | 2456 | 0.5 | 6 | 3.0 | |
| **4** | MgCl2 | NA | 0.0 | 0 | 0.0 | |

| **Set 3** | **Reagent** | **Lot #** | **Volume (uL)** | **Reaction No.** | **Total Amount** | **Target** |
|---|---|---|---|---|---|---|
| **1** | H2O | 4532 | 16.5 | 4 | 66.0 | (6) Geo |
| **2** | P/P Working Stock | 020705 | 3.5 | 4 | 14.0 | |
| **3** | SmartMix (Bead) | 2456 | 0.5 | 4 | 2.0 | |
| **4** | MgCl2 | NA | 0.0 | 0 | 0.0 | |

| | | | | | | |
|---|---|---|---|---|---|---|
| *Add MgCl2 as needed per target subtract volume used from water added to maintain a 20uL reaction. Add 20 uL of Master Mix to each tube and then add 5.0 uL of template for a total volume of 25.0 uL. | | | | | | |

**III. Sample Position**

| **Position** | **Sample** | **Extract Date** | **Position** | **Sample** | **Extract Date** |
|---|---|---|---|---|---|
| **I** | (3)6532 | 062405 | **9** | (2) Positive Control | 052705 |
| **2** | (3) 6789 | 062405 | **10** | (2) Negative Control | 052005 |
| **3** | (3) 7546 | 062405 | **11** | (6) 6532 | 062405 |
| **4** | (3) Positive Control | 052705 | **12** | (6) 6789 | 062405 |
| **5** | (3) Negative | 052005 | **13** | (6) 7546 | 062405 |
| | Control | | | | |
| **6** | (2) 6532 | 062405 | | | **14** |
| **7** | (2)6789 | 062405 | | | **15** |
| **8** | (2) 7546 | 062405 | | | **16** |

**Data** Analysis**:** After the run was completed the results were analyzed by reviewing each site in the results table. If a specific sample tested was registered as positive by the software there was a positive in the results column for that sample. There was also a crossing point registered in the Ct column for that sample. A sample was analyzed as positive by the software if the curve breaks the fluorescence baseline threshold before the end of 40 of the 45 cycles and negative if it did not break the fluorescence baseline threshold before the end of 40 of the 45 cycles.

**Results Interpretation:** A positive result was defined as any amplification observed crossing the fluorescence baseline threshold between cycles 1 and 40 of the real-time PCR run. A negative result was defined as no amplification observed crossing the fluorescence baseline threshold between cycles 1 and 40 of the PCR run. An equivocal result was defined as amplification observed crossing the fluorescence baseline threshold between cycles 40 and 45, a control out of range, or questions regarding sample integrity. A positive control was defined as a control that was positive for the target being tested and showed that the assay would detect the presence of target DNA and that there was not PCR inhibition. (Note: a sample that showed amplification for a target when the positive control was negative was reported as a positive result). A negative control was defined as a control that was negative for the target being tested and showed that the reagents or the sample were not contaminated with the target prior to the testing of the sample. An internal control was a control used to show that the extraction process was working for the purification of nucleic acid from the clinical specimen and that a negative result was truly negative and not due to an issue associated with the extraction. (Note: the internal control must be positive for any sample to be reported as negative for a target.)

**Table: Results Interpretation**

| | | | | |
|---|---|---|---|---|
| **Reportable** | **Crossing** | **Positive** | **Negative** | **Internal** |

| **Result** | **Point** | **Control** | **Control** | **Control** |
|---|---|---|---|---|
| Positive Result | <40 | (+) | (-) | (+) |
| Positive Result | <40 | (-) | (-) | (+) |
| Positive Result | <40 | (+) | (-) | (-) |
| Positive Result | <40 | (-) | (-) | (-) |
| Negative Result | (-) | (+) | (-) | (+) |
| Negative Result | (-) | (+) | (+) | (+) |
| Negative Result | (-) | (-) | (+) | (+) |

| **Unreportable Result** | **Crossing Point** | **Positive Control** | **Negative Control** | **Internal Control** |
|---|---|---|---|---|
| Positive Result | <40 | (+) | (+) | (+) |
| Positive Result | <40 | (-) | (+) | (+) |
| Positive Result | <40 | (+) | (+) | (-) |
| Positive Result | <40 | (-) | (+) | (-) |
| Negative Result | (-) | (-) | (-) | (+) |
| Negative Result | (-) | (+) | (-) | (-) |
| Negative Result | (-) | (+) | (+) | (-) |

### EXAMPLE 28

### COMPARING A SINGLE STEP PCR METHOD TO A TWO-STEP PCR METHOD ON HUMAN SERUM SAMPLES

In the following Example, RTL stands for Real Time Labs PCR results. This is a real-time PCR using the specially designed second set of primers and probes only. Dart PCR stands for Real-time Polymerase Chain Reaction (DART PCR) refers to the two-step PCR method as described herein. The methods used to obtain the data below are described in Example 2, 3, 4, and 5. The samples were of human serum.

| **2014 University of Florida Serum Samples** | | | | | | | |
|---|---|---|---|---|---|---|---|
| **Sample ID** | **RTL PCR Results** | **Dart PCR Results** | **RTLPCR Results** | **Dart PCR Results** | **RTL PCR Results** | **Dart PCR Results** | |
| | **A fumigatus** | **A fumigatus** | **A flavus** | **A flavus** | **A niger** | **A niger** | **Comment** |
| 100-035 | Not Detected | Positive | Not Detected | Not Detected | Not Detected | Positive | Proven Aspergillus fumigatus |
| 100-040 | Not Detected | Probable | Not Detected | Not Detected | Not Detected | Not Detected | N/A |
| 100-082 | Not Detected | Not Detected | Not Detected | Not Detected | Not Detected | Not Detected | Proven Aspergillus fumigatus |
| 100-081 | Not Detected | Positive | Not Detected | Not Detected | Not Detected | Not Detected | N/A |
| 100-106 | Not Detected | Not Detected | Not Detected | Not Detected | Not Detected | Not Detected | Probable Aspergillus Species |
| 100-109 | Not Detected | Not Detected | Not Detected | Positive | Not Detected | Not Detected | N/A |
| 100-124 | Not Detected | Not Detected | Not Detected | Not Detected | Not Detected | Not Detected | Probable Aspergillus Species |
| 100-128 | Not Detected | Positive | Not Detected | Not Detected | Not Detected | Positive | N/A |
| 100-151 | Not Detected | Not Detected | Not Detected | Positive | Not Detected | Not Detected | Probable Aspergillus Species |
| 100-152 | Not Detected | Not Detected | Not Detected | Not Detected | Not Detected | Not Detected | N/A |
| 100-155 | Not Detected | Positive | Not Detected | Not Detected | Not Detected | Not Detected | Probable Aspergillus Species |
| 100-156 | Not Detected | Not Detected | Not Detected | Not Detected | Not Detected | Not Detected | N/A |

The data illustrate that the two-step PCR method was able to detect fungal DNA where a single step PCR method could not. The initial amplification step provides more sensitivity to the assay which allowed for detection of fungal targets previously undetected.

### EXAMPLE 29

### COMPARING A SINGLE STEP PCR METHOD TO A TWO-STEP PCR METHOD ON PATIENT SAMPLES

In the following Example, RTL stands for Real Time Labs PCR results. This is a real-time PCR using the specially designed second set of primers and probes only. Dart PCR stands for Real-time Polymerase Chain Reaction (DART PCR) refers to the two-step PCR method as described herein. The methods used to obtain the data below are described in Example 2, 3,4, 5, and 27. The samples were from human patients.

| | **Aspergillus Samples** | **Clinical Samples** | | |
|---|---|---|---|---|
| | **2012 (RTL Accession)** | **Sample Type** | **RTL PCR Results** | **DART PCR Results** |
| **1** | 121416 | Tissue | Not Detected | A flavus |
| **2** | 124598 | BAL | Not Detected | Not Detected |
| **3** | 124601 | Tissue | Not Detected | Not Detected |
| **4** | 124592 | Tissue | Not Detected | Not Detected |
| **5** | 122162 | Fungal Isolate | Not Detected | Not Detected |
| **6** | 122341 | Tissue | Not Detected | A fumigatus |
| **7** | 124314 | Sputum | Not Detected | Not Detected |

| | **2013 (RTL Accession)** | | | |
|---|---|---|---|---|
| **8** | 127031 | Tissue | Not Detected | Not Detected |
| **9** | 131415 | Tissue | Not Detected | A terreus |
| **10** | 132172 | Nasal Wash | Not Detected | Not Detected |

| | **2014 (RTL Accession)** | | | |
|---|---|---|---|---|
| **11** | 142975 | Tissue | Not Detected | A terreus |
| **12** | 142976 | Tissue | Not Detected | Not Detected |
| **13** | 142977 | Tissue | Not Detected | Not Detected |
| **14** | 144744 | Tissue | Not Detected | Not Detected |

| | **2015 (RTL Accession)** | | | |
|---|---|---|---|---|
| **15** | 153775 | BAL | Not Detected | Not Detected |
| **16** | 157251 | Tissue | Not Detected | Not Detected |
| **17** | 138789 | Whole Blood | Not Detected | Not Detected |
| **18** | 163014 | Nasal Wash | Not Detected | Not Detected |
| **19** | 160271 | Tissue | Not Detected | Not Detected |
| **20** | 159348 | Tissue | Not Detected | Not Detected |
| **21** | 156473 | Tissue | Not Detected | Not Detected |
| **22** | 162119 | Whole Blood | Not Detected | Not Detected |
| **23** | 163871 | Nasal Wash | Not Detected | Not Detected |

| | **2016 (RTL Accession)** | | | |
|---|---|---|---|---|
| **24** | 162026 | BAL | Not Detected | A fumigatus |
| **25** | 166399 | Nasal Wash | Not Detected | Not Detected |

| | **Candida Samples** | **Clincal Samples** | | |
|---|---|---|---|---|
| | **2012(RTL Accession)** | | | |
| **1** | 120468 - 2012-1 | Urine | Not Detected | C albicans, C glabrata |
| **2** | 122339 - 2012-64 | Tissue | Not Detected | C parapsilosis |
| **3** | 124321 - 2012-79 | Sputum | Not Detected | Not Detected |

| | **2013(RTL Accession)** | | | |
|---|---|---|---|---|
| **4** | 1258533 - 2013-17 | Urine | Not Detected | C parapsilosis |
| **5** | 127635 - 2013-51 | Urine | Not Detected | C parapsilosis |
| **6** | 134356 - 2013-55 | Urine | Not Detected | C parapsilosis, C kruseii |

| | **2014(RTL Accession)** | | | |
|---|---|---|---|---|
| **7** | 133886 - 2014-1 | Urine | Not Detected | C parapsilosis |
| **8** | 126773 - 2014-15 | Urine | Not Detected | C parapsilosis |
| **9** | 144956 - 2014-21 | Urine | Not Detected | C albicans |
| **10** | 144955 - 2014-41 | Urine | Not Detected | C parapsilosis |

| | **2015(RTL Accession)** | | | |
|---|---|---|---|---|
| **11** | 150827 - 2015-3 | Urine | Not Detected | C albicans, C parapsilosis |
| **12** | 163352 - 2015-52 | Tissue | Not Detected | C parapsilosis, C kruseii |
| **13** | 156078 - 2015-47 | Urine | Not Detected | C parapsilosis |
| **14** | 163698 - 2015-60 | Tissue | Not Detected | Not Detected |
| **15** | 133742 - 2015-63 | Urine | Not Detected | Not Detected |
| **16** | 164464 - 2015-64 | Urine | Not Detected | Not Detected |
| **17** | 163791 - 2015-70 | Urine | Not Detected | C parapsilosis |

| | **2016(RTL Accession)** | | | |
|---|---|---|---|---|
| **18** | 154204 - 2016-07 | Urine | Not Detected | C parapsilosis |
| **19** | 165667 - 2016-10 | Urine | Not Detected | C parapsilosis |
| **20** | 166123 - 2016-12 | Urine | Not Detected | Not Detected |
| **21** | 167007 - 2016-18 | Urine | Not Detected | C parapsilosis |
| **22** | 167005 - 2016-21 | Urine | Not Detected | Not Detected |

The table above demonstrates that the two-step PCR method was able to detect fungal DNA in patient samples where a single primer and probe real-time PCR analysis could not. The two-step method provided more sensitivity to detect the presence of fungal DNA that was undetectable using a single step PCR method. The two-step method was able to detect fungal DNA regardless of sample type including fluid or tissue.

## Claims

1. A method of identifying a specific fungal species in a patient tissue or a patient body fluid, the method comprising:
extracting and recovering fungal DNA of the fungal species from the patient tissue or the patient body fluid,
amplifying the fungal DNA using a first set of primers to produce an amplicon,
amplifying the amplicon using a second set of primers,
hybridizing an isolated probe to the amplicon to identify the specific fungal species, wherein the probe is labeled with at least one fluorescent dye, and
identifying the specific fungal species.

2. A method of determining if a patient is at risk for or has developed a disease state related to a fungal infection, the method comprising:
extracting and recovering fungal DNA of the fungal species from the patient tissue or the patient body fluid,
amplifying the fungal DNA using a first set of primers to produce an amplicon,
amplifying the amplicon using a second set of primers,
hybridizing an isolated probe to the amplicon to identify the specific fungal species, wherein the probe is labeled with at least one fluorescent dye,
identifying the specific fungal species to determine if the patient is at risk for or has developed the disease state related to a fungal infection.

3. The method of claim 1 or 2 wherein the amplifying steps are performed with primers that hybridize to the fungal DNA or to the amplicon.

4. The method of claim 1 or 2 wherein the body fluid is selected from the group consisting of urine, nasal secretions, nasal washes, bronchial lavages, bronchial washes, spinal fluid, sputum, gastric secretions, seminal fluid, other reproductive tract secretions, lymph fluid, whole blood, blood from a blood card, serum, buffy coat, and plasma.

5. The method of claim 1 or 2 wherein the fungal DNA or the amplicon is amplified using PCR, preferably the PCR is real-time PCR.

6. The method of claim 1 or 2 wherein the fungal species is selected from the group consisting of *Aspergillus niger, Aspergillus flavus, Aspergillus fumigatus, Aspergillus terreus, Candida albicans, Candida auris, Candida tropicalis, Candida glabrata, Candida krusei, and Candida parapsilosis.*

7. The method of claim 1 or 2 wherein the first set of primers is selected from the group consisting of SEQ ID NOS:1 and 2, SEQ ID NOS:3 and 4, SEQ ID NOS:5 and 6, SEQ ID NOS:7 and 8, SEQ ID NOS:9 and 10, SEQ ID NOS:11 and 12, SEQ ID NOS:13 and 14, SEQ ID NOS:15 and 16, SEQ ID NOS: 17 and 18, and SEQ ID NOS: 55 and 56.

8. The method of claim 1 or 2 wherein the second set of primers is selected from the group consisting of SEQ ID NOS:20 and 21, SEQ ID NOS:23 and 24, SEQ ID NOS:26 and 27, SEQ ID NOS:29 and 30, SEQ ID NOS:32 and 33, SEQ ID NOS:35 and 36, SEQ ID NOS:38 and 39, SEQ ID NOS:41 and 42, SEQ ID NOS:44 and 45, SEQ ID NOS:47 and 48, and SEQ ID NOS: 58 and 59.

9. The method of claim 1 or 2 wherein the probe is selected from the group consisting of SEQ ID NOS: 19, 22, 25, 28, 31, 34, 37, 40, 43, 46, and 57.

10. The method of claim 1 or 2 wherein the fungal DNA or the amplicon is from internal transcribed spacer regions of nuclear ribosomal DNA.

11. The method of claim 1 or 2 wherein the at least one fluorescent dye is selected from the group consisting of a fluorescence resonance energy transfer pair, FAM, dCAL FLUOR Orange 560, and BHQ.

12. The method of claim 1 or 2 further comprising quantifying the fungal DNA.

13. The method of claim 1 or 2 wherein the method can be used to detect 0.1 ng of the fungal DNA.

14. The method of claim 1 further comprising identifying a mycotoxin in the patient tissue or body fluid.

15. The method of claim 1 wherein the fungal DNA is amplified in a separate reaction vessel than the amplicon.

16. The method of claim 1 wherein the fungal DNA is detected by amplifying the fungal DNA using the first set of primers to produce the amplicon, and amplifying the amplicon using the second set of primers, but is not detected using a single real-time PCR amplification step.

17. A purified nucleic acid with a sequence selected from SEQ ID NOS: 1 to 18, 55, and 56, a complement of a sequence selected from SEQ ID NOS: 1 to 18, 55, and 56, in combination with fluorescently labeled target DNA.
